(19) Europäisches Patentamt
European Patent Office
Office européen des brevets

(11) EP 3 412 775 A1

(12) EUROPEAN PATENT APPLICATION
published in accordance with Art. 153(4) EPC

(43) Date of publication:
**12.12.2018 Bulletin 2018/50**

(21) Application number: **17747843.5**

(22) Date of filing: **06.02.2017**

(51) Int Cl.:
*C12N 15/86* (2006.01) *C07K 14/47* (2006.01)
*C12N 9/10* (2006.01) *C12N 9/78* (2006.01)
*C12N 5/0775* (2010.01) *A61K 35/28* (2015.01)
*A61K 48/00* (2006.01)

(86) International application number:
**PCT/KR2017/001308**

(87) International publication number:
**WO 2017/135800 (10.08.2017 Gazette 2017/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.02.2016 KR 20160015103**

(71) Applicant: **SIbigen Inc.**
**Seongnam-si, Gyeonggi-do 13488 (KR)**

(72) Inventors:
- **SUNG, Young Chul**
**Seoul 04385 (KR)**
- **LEE, Soon Min**
**Seoul 06762 (KR)**
- **KIM, Hey-yon**
**Seoul 05578 (KR)**

(74) Representative: **Grünecker Patent- und Rechtsanwälte PartG mbB**
**Leopoldstraße 4**
**80802 München (DE)**

# (54) MESENCHYMAL STEM CELL EXPRESSING TRAIL AND CD, AND USE THEREOF

(57) The present invention relates to: a recombinant lentiviral vector comprising a gene encoding a TRAIL protein and a CD protein; and a cell that is transfected with the lentivirus produced by using the vector. A host cell transfected with the recombinant lentivirus of the present invention maintains a high cell proliferation rate and overexpresses a TRAIL protein and a CD protein. Thus, a mesenchymal stem cell transfected with the lentivirus may be usefully employed as a cell therapeutic agent.

[Figure 6]

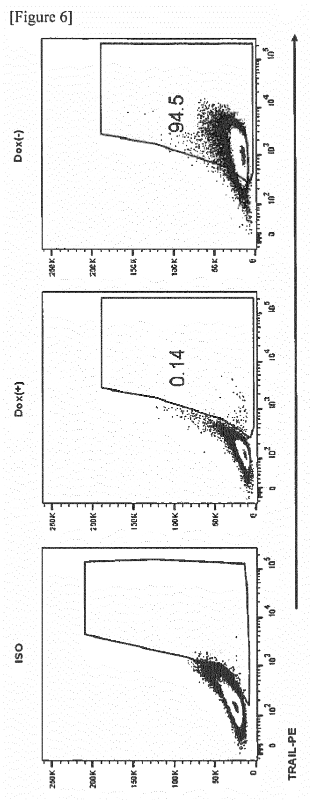




Printed by Jouve, 75001 PARIS (FR)

## Description

### Technical Field

The present invention relates to a recombinant lentiviral vector comprising a gene encoding a TRAIL (TNF-related apoptosis-inducing ligand) protein and a CD (cytosine deaminase) protein, and a cell transfected with the lentivirus produced by using the vector.

### Background Art

Therapies utilizing cells are being developed globally, and especially, the stem cell therapy market is showing a steady upward trend with an annual average growth rate of 11.7%.

Mesenchymal stem cells (MSCs), which are adult stem cells, are multipotent cells that can differentiate into bones, cartilages, muscles, fats, and fibroblasts, etc. In addition, MSCs can be obtained from various adult tissues such as bone marrow, umbilical cord blood, and fats, relatively easily. MSCs are characterized by their ability to migrate to the site of inflammation or injury, which is also a great advantage as a delivery vehicle for delivering a therapeutic drug. Further, the immune function of the human body can be regulated by inhibiting the functions of immune cells such as T cells, B cells, dendritic cells and natural killer cells. Additionally, MSCs have an advantage that it can be cultured relatively easily *in vitro,* and thus studies for using MSCs as a cell therapeutic agent are being actively carried out.

However, despite such advantages of MSCs, there are following problems in producing MSCs that can be used clinically as a cell therapeutic agent. First, since there is a limitation in the proliferation of MSCs, it is difficult to produce them in large quantities. Second, since the MSCs obtained are heterogenous, it is difficult to maintain the same effect in every production. Third, the use of MSCs alone is not therapeutically effective.

On the other hand, Korean Patent No. 1585032 discloses a cell therapeutic agent containing mesenchymal stem cells cultured in a hydrogel. The above document provides a composition that can be administered directly by shortening the pretreatment process in the step of isolating mesenchymal stem cells for use as a cell therapeutic agent. However, the problems of the mesenchymal stem cells described above and the method for solving the problems are not mentioned at all. Therefore, it is necessary to study mesenchymal stem cells which can be useful as a cell therapeutic agent.

### Disclosure of Invention

### Technical Problem

An object of the present invention is to provide a recombinant lentivirus comprising a gene encoding a TRAIL protein and a CD protein and a host cell transfected with the above recombinant lentivirus.

Another object of the present invention is to provide a pharmaceutical composition comprising the above recombinant lentivirus or host cell.

### Solution to Problem

In accordance with one object of the present invention, there is provided a recombinant lentiviral vector comprising a gene encoding a TRAIL protein and a CD protein.

Further, in accordance with another object of the present invention, there is provided a recombinant lentivirus comprising a gene encoding a TRAIL protein and a CD protein.

Further, in accordance with another object of the present invention, there is provided a host cell transfected with the above recombinant lentivirus.

Further, in accordance with another object of the present invention, there is provided a pharmaceutical composition for preventing or treating cancer comprising the above recombinant lentivirus as an active ingredient.

Further, in accordance with another object of the present invention, there is provided a pharmaceutical composition for preventing or treating cancer comprising the above host cell as an active ingredient.

### Advantageous Effects of Invention

A host cell transfected with a recombinant lentivirus comprising a gene encoding a TRAIL protein and a CD protein of the present invention expresses a TRAIL protein and a CD protein and maintains a high cell proliferation rate. In addition, abnormal differentiation can be inhibited and the possibility of tumor formation can be blocked, indicating high safety. Therefore, the lentivirus or the host cell can be useful as a cell therapeutic agent.

**Brief Description of Drawings**

Fig. 1 is a graph comparing cell proliferation rates of immortalized MSCs and non-immortalized MSCs:

imMSC: immortalized MSC;
MSC: non-immortalized MSC;
X axis: incubation period; and
Y axis: cumulative population doubling level (PDL).

Fig. 2 is a schematic representation of the structure of a gene construct inserted into a pBD-4 lentiviral vector:

TRE: a promoter comprising tetracycline response elements;
TRAIL: TNF-associated apoptosis-inducing ligand;
IRES: internal ribosome entry site; and
CD: CD protein.

Fig. 3 compares the expressions of the surface antigen proteins CD90, CD44, CD105, CD73, etc., which are inherent characteristics of MSC, of the MSC after gene manipulation of the BM-03 cell line and of the bone marrow-derived MSC.

Fig. 4 shows the differentiation ability of the BM-03 cell line after gene manipulation, in which adipogenesis, osteogenesis, and chondrogenesis of the BM-03 cell line after gene manipulation were identified.

Fig. 5 shows the results of detection for transgene(TRAIL and CD) in BM-03, a deposited strain. Lane 1 shows a marker, lane 2 shows a negative control, lane 3 shows a positive control, and lanes 4 to 6 show BM-03.

Fig. 6 shows doxycycline dependent TRAIL expression using FACS.

Fig. 7 shows cell death by treating mesenchymal stem cells expressing a TRAIL protein and a CD protein with 5FC, which was examined by FACS.

Fig. 8 is a graph showing the PDL of BM-03 cells obtained by subculture.

Fig. 9 shows the results of analysis of the karyotype of the BM-03 cell into which the gene was introduced.

**Best Mode for Carrying out the Invention**

Hereinafter, the present invention will be described in detail.

The present invention provides a recombinant lentiviral vector comprising a gene encoding a TRAIL protein and a CD protein.

As used herein, the term "TNF-related apoptosis-inducing ligand (hereinafter, referred to as TRAIL)" protein belongs to the type 2 transmembrane cytokine in the TNF family. The TRAIL selectively induces apoptosis of transformed cells as one of suicide genes. Specifically, TRAIL binds to death receptor-4 (DR-4), DR-5, decoy receptor or decoy receptor-2 present on the cell surface to activate the apoptosis signal transduction system. TRAIL is not toxic to normal cells, and is known to specifically induce apoptosis of cancer cells only.

A TRAIL protein according to the present invention may be a human-derived protein. The TRAIL protein is present in the form of a homotrimer capable of binding to three receptors. The TRAIL protein of the present invention may be a polypeptide having the amino acid sequence of SEQ ID NO: 1. The TRAIL protein may have about 70%, 80%, 90%, 95% or higher homology with the amino acid sequence of SEQ ID NO: 1. Meanwhile, the gene encoding the TRAIL protein may be a polynucleotide having the nucleotide sequence of SEQ ID NO: 2. In addition, the nucleotide sequence encoding the TRAIL protein may have about 70%, 80%, 90%, 95% or higher homology with the nucleotide sequence of SEQ ID NO: 2.

As used herein, the term "CD protein" is a cytosine deaminase protein, and may be in the form of a fusion protein in which CD and uracil phosphoribosyltransferase (UPRT) are linked, and the term "CD protein" as used herein, can be used interchangeably with "CD::UPRT".

The apoptosis of a cell expressing the above CD protein is induced by converting 5-fluorocytosine (5FC) into 5-fluorouracil (5FU) having strong cytotoxicity. Therefore, the apoptosis of the cell containing a lentiviral vector comprising a CD protein gene can be induced by treatment with 5FC.

The sequence of the gene encoding a CD protein according to the present invention is a codon-optimized sequence by fusing the FCY1 gene encoding the CDase of *Saccharomyces cerevisiae* and the FUR1Δ105 gene encoding the UPRTase in which 35 amino acids from the N-terminal are deleted. Such sequences may be those described in U.S. Patent No. 5,338,678, International Patent Publication No. WO 96/16183, and International Patent Publication No. WO 99/54481. According to one embodiment, the CD protein may be a polypeptide having the amino acid sequence of SEQ ID NO: 3. In addition, the CD protein may have about 70%, 80%, 90% or 95% or higher homology with the amino acid sequence of SEQ ID NO: 3. Meanwhile, the gene encoding the CD protein may be a polynucleotide having the nucleotide sequence of SEQ ID NO: 4. In addition, the nucleotide sequence encoding the CD protein may have about 70%, 80%, 90% or 95% or higher homology with the nucleotide sequence of SEQ ID NO: 4.

The term "lentiviral vector" as used herein is a kind of retroviruses, which is a vector in the form of single stranded RNA, and may also be referred to as "lentivirus transfer vector". The lentiviral vector can be inserted into the genomic DNA of a target cell of infection to stably express the gene, and can transfer the gene to the mitotic and non-mitotic cells. Since the vector does not induce the immune response of a human body, its expression is continuous. In addition, there is an advantage that genes of a large size can be delivered as compared to an adenovirus vector which is a conventional virus vector.

The lentiviral vector may further comprise a gene encoding a thymidine kinase (TK) protein. The TK protein is an enzyme that catalyzes the thymidyl acid production reaction by binding phosphoric acid at the γ-position of ATP to thymidine, whereby thymidine is transformed into a triphosphate form. The modified thymidine cannot be used for DNA replication, and is known to induce death of cells containing it. The TK protein for use herein may be one of any known sequences. According to one embodiment, the TK protein may be a polypeptide having the amino acid sequence of SEQ ID NO: 5. Meanwhile, the gene encoding the TK protein may be a polynucleotide having the nucleotide sequence of SEQ ID NO: 6.

The recombinant lentiviral vector of the present invention can comprise 1 or 2 promoters. The promoter may be a cytomegalovirus (CMV), respiratory syncytial virus (RSV), human elongation factor-1 alpha (EF-1α) or tetracycline response elements (TRE) promoter. According to one embodiment, the recombinant lentiviral vector can regulate the expression of a TRAIL or a CD protein by one promoter. The promoter can be operably linked to a gene encoding a protein to be expressed.

According to one embodiment, the TRAIL and CD proteins may be linked to a TRE promoter. The TRE promoter can activate the transcription of the gene linked to the promoter by the tetracycline transactivator (tTA) protein. Specifically, the tTA protein binds to the TRE promoter and activates transcription when tetracycline or doxycycline is not present, whereas it cannot bind to the TRE promoter and activate the transcription when tetracycline or doxycycline is present. Thus, the expression of a TRAIL or a CD protein can be regulated by the addition or depletion of tetracycline or doxycycline.

The term "operably linked" means that a particular polynucleotide is linked to another polynucleotide so that it can conduct its function. The expression that a gene encoding a specific protein is operatively linked to a promoter implies that it is linked such that the gene can be transcribed into mRNA by the action of the promoter and translated into a protein.

When two or more genes are linked such that they are transcribed by one promoter in a lentiviral vector of the present invention, the vector may contain the ribosome entry site (IRES) so that each protein can be expressed from the single transcript.

The term "internal ribosome entry site (IRES)" refers to a nucleic acid sequence that functions to enable translation from an intermediate region of a transcript instead of the 5'-cap structure during eukaryotic protein synthesis. By using IRES, multiple proteins conducting different functions can be produced from a single transcript. According to one embodiment, the recombinant lentiviral vector of the present invention, in which a TRAIL protein and a CD protein are linked via IRES, can be transcribed into a single transcript, and then each protein can be produced therefrom.

The present invention provides a recombinant lentivirus comprising a gene encoding a TRAIL protein and a CD protein.

The recombinant lentivirus may be obtained by the steps of transforming a host cell with a lentiviral vector of the present invention, a packaging plasmid and an envelope plasmid; and isolating the lentivirus from the transformed host cell.

The terms "packaging plasmid" and "envelope plasmid" may provide helper constructs(e.g., plasmids or separated nucleic acid) for producing lentiviruses from the lentiviral vectors of the present invention for effective transfection. Such constructs contain useful elements for preparing and packaging lentiviral vectors in host cells. The above elements include a structural protein such as a gag precursor; a processing protein such as a pol precursor; protease; coat protein; and expression and regulatory signal necessary to prepare proteins and produce lentiviral particles in the host cell, etc.

For production of the recombinant lentivirus, Lenti-X Lentiviral Expression System provided by Clontech Laboratories Inc., a packaging plasmid (e.g., pRSV-Rev, psPAX, pCI-VSVG, pNHP, etc.) or an envelope plasmid (e.g.,

pMD2.G, pLTR-G, pHEF-VSVG, etc.) provided by Addgene can be used.

Further, the present invention provides a host cell transfected with the above recombinant lentivirus.

The term "transfection" refers to the delivery of a gene loaded in a recombinant lentiviral vector through viral infection.

A host cell according to the present invention may be a human embryonic stem cell (hES), a bone marrow stem cell (BMSC), a mesenchymal stem cell (MSC), a human neural stem cell (hNSCs), a limbal stem cell, or an oral mucosal epithelial cell. According to an embodiment of the present invention, the host cell may be a mesenchymal stem cell.

The term "mesenchymal stem cell (MSC)" refers to a multipotent stromal cell capable of differentiating into various cells including osteocytes, chondrocytes and adipocytes. Mesenchymal stem cells can differentiate into the cells of specific organs such as a bone, a cartilage, a fat, a tendon, a nervous tissue, fibroblasts and myocytes. These cells can be isolated or purified from adipose tissues, bone marrows, peripheral blood, umbilical cord blood, periosteum, dermis, mesodermal-derived tissues, and the like.

The host cell may be prepared by the following method:

1) primary infection of host cells with the lentiviruses comprising hTERT and c-myc genes;
2) secondary infection of the primary-infected host cells with the lentiviruses comprising a tTA gene; and
3) tertiary infection of the secondary-infected host cells with the lentiviruses comprising a TRAIL gene and a CD gene.

In the step 1), hTERT and c-myc are genes that immortalize host cells. Genes known as immortalizing genes other than hTERT and c-myc can also be used. According to one embodiment, the hTERT and c-myc proteins may be polypeptides having the amino acid sequences of SEQ ID NO: 9 and SEQ ID NO: 7, respectively. Meanwhile, the genes coding for the hTERT and c-myc proteins may be polynucleotides having the nucleotide sequences of SEQ ID NO: 10 and SEQ ID NO: 8, respectively.

In the step 2), tTA is a gene capable of regulating the expression of a target protein, which means tetracycline transactivator. The Tet-off system as used herein can regulate the expression of a target protein depending on the presence or absence of tetracycline or doxycycline as described above.

The tertiary infection of the step 2) can be carried out using a vector of the present invention which contains both TRAIL gene and CD gene in a single vector. However, in another aspect of the present invention, the TRAIL gene and CD genes can be prepared as each gene construct and inserted into two lentiviral vectors, respectively. That is, a lentiviral vector into which a gene construct prepared such that a gene encoding a TRAIL protein can be expressed is inserted, and a lentiviral vector into which a gene construct prepared such that a gene encoding a CD protein can be expressed is inserted can be used for the tertiary infection.

The prepared cells by the above method were designated as BM-03 and deposited on Jan. 6, 2017 with the deposit number KCTC 13182BP at Korean Collection for Type Cultures at Korea Research Institute of Bioscience & Biotechnology (KRIBB).

The present invention provides a pharmaceutical composition for preventing or treating cancer, comprising the above recombinant lentivirus or host cell as an active ingredient.

The cancer refers to a general cancer encompassing any blood cancer and solid cancer. Examples of cancer may include gastric cancer, colon cancer, breast cancer, lung cancer, non-small cell lung cancer, bone cancer, pancreatic cancer, skin cancer, head and neck cancer, melanoma, uterine cervix cancer, ovarian cancer, rectal cancer, endometrial cancer, Hodgkin’s disease, a brain tumor, sarcoma, esophageal cancer, small bowel cancer, thyroid cancer, prostate cancer, leukemia, lymphoma, bladder cancer, a central nervous system tumor, a spinal cord tumor, etc.

The pharmaceutical composition is a kind of cell therapeutic agents, and may further comprise a pharmaceutically acceptable carrier. The carrier may be one generally used in the preparation of medicines, which may include lactose, dextrose, sucrose, sorbitol, mannitol, starches, gum acacia, calcium phosphate, alginates, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like.

In addition, the pharmaceutical compositions of the present invention may further comprise a pharmaceutically acceptable additive, which be selected from the group consisting of a lubricant, a wetting agent, a sweetening agent, a flavoring agent, an emulsifying agent, a suspending agent, a preservative and a combination thereof.

The carrier may be comprised in an amount of about 1 % to about 99.99% by weight, preferably about 90% to about 99.99% by weight, based on the total weight of the pharmaceutical composition of the present invention, and the pharmaceutically acceptable additive may be comprised in an amount of about 0.1% to about 20% by weight.

The pharmaceutical composition may be prepared in a unit dosage form by formuling with a pharmaceutically acceptable carrier and excipient according to a conventional method, or may be prepared by filling into a multi-dose container. Herein, the formulation may be in the form of a solution, a suspension, a syrup or an emulsion in oil or aqueous media, or in the form of an extract, powders, a powdered drug, granules, tablets or capsules, and may additionally contain a dispersing or stabilizing agent.

The present invention also provides a method for preventing or treating cancer as described above, comprising the step of administering a pharmaceutical composition of the present invention to a subject.

The subject may be a mammal, particularly a human. The administration route and dosage of the pharmaceutical composition may be adjusted in various ways and amounts for administration to a subject depending on the condition of a patient and the presence of side effects, and the optimal administration method and dosage may be selected by a person skilled in the art in a suitable range. In addition, the pharmaceutical composition may be administered in combination with other drugs or physiologically active substances known to have a therapeutic effect on a disease to be treated, or may be formulated in a form of combination formulation with other drugs.

When the pharmaceutical composition is administered parenterally, examples of the administration include subcutaneous, ocular, intraperitoneal, intramuscular, oral, rectal, intraorbital, intracerebral, intracranial, intraspinal, intraventricular, intrathecal, intranasal, intravenous administration.

The above administration may be administered for one or more times, one to three times, specifically in two divided doses. In the case of repeated administrations, they can be administered at the interval of 12 to 48 hours, 24 to 36 hours, and more specifically, at the interval of 24 hours. In the case of lentiviruses, the administration may be conducted in an amount of 1.0 x $10^6$ to 1.0 x $10^{12}$ TU, specifically 1.0 x $10^8$ to 1.0 x $10^{10}$ TU for adults. On the other hand, in the case of cells, the administration may be conducted in an amount of 1.0 x $10^5$ to 1.0 x $10^{11}$ cells, specifically 1.0 x $10^7$ to 1.0 x $10^9$ cells for adults. When the dose is high, the administration may be conducted several times a day.

## Mode for the Invention

Hereinafter, the present invention is described in detail with reference to the following examples. However, the following examples are intended to illustrate the present invention, and the present invention is not limited thereto.

## Example 1. Preparation of immortalized mesenchymal stem cells (MSCs)

### 1-1. Preparation of lentiviral vectors containing immortalized gene

In order to immortalize MSCs, lentiviral vectors respectively containing c-Myc and hTERT, which are immortalized genes, were prepared. Herein, a gene construct expressing the tTA protein was inserted together to use the Tet-off system.

First, a pBD lentiviral vector was prepared by substituting the EF promoter in the expression cassette of the pWPT vector (Addgene, USA) with the CMV promoter, and adding the RSV promoter to the downstream thereof.

The c-Myc gene (SEQ ID NO: 8) and thymidine kinase (TK) gene (SEQ ID NO: 6) were linked via IRES and inserted into the pBD lentiviral vector so that the expression can be regulated by the CMV promoter. The constructed vector was designated as pBD-1.

On the other hand, the hTERT gene (SEQ ID NO: 10) was inserted into the pBD lentiviral vector such that the expression can be regulated by the CMV promoter. A gene having resistance to zeomycin (ZeoR; SEQ ID NO: 16) was inserted thereto such that the expression can be regulated by the RSV promoter. The constructed vector was designated as pBD-2.

In addition, a tTA (tetracycline transactivator) gene (SEQ ID NO: 12) was inserted into the pBD lentiviral vector such that the expression can be regulated by the CMV promoter. A gene having resistance to puromycin (PuroR; SEQ ID NO: 14) was inserted thereto such that the expression can be regulated by the RSV promoter. The constructed vector was designated as pBD-3.

### 1-2. Production of lentiviruses containing immortalized gene

Using the lentiviral vectors constructed in Example 1-1, lentiviruses containing immortalized genes were produced by the following method.

First, Lenti-X cells (Clontech Laboratories, USA) were cultured in a 150 mm dish using DMEM supplemented with 10% fetal bovine serum. Meanwhile, lentiviral vectors were extracted and quantified from DH5$\alpha$ *E. coli* cells using EndoFree Plasmin Maxi Kit (Qiagen, USA).

The cultured Lenti-X cells were washed with PBS, and then 3 ml of TrypLE ™ Select CTS™ (Gibco, USA) was added thereto. The cells were left at 37°C for about 5 minutes, and then their detachment was verified. The detached cells were neutralized by adding 7 ml of DMEM supplemented with 10% fetal bovine serum thereto. The neutralized cells were collected in a 50 ml tube and centrifuged at 1,500 rpm for 5 minutes. The resultant supernatant was removed and the cells were resuspended by adding 10 ml of DMEM supplemented with 10% fetal bovine serum thereto. The suspended cells were counted with a hematocytometer and then dispensed into a 150 mm dish in an amount of 1.2 x $10^7$ cells. When the dispensed cells were cultured to a cell saturation of about 90%, the cells were transformed with a

mixture of 12 μg of lentiviral vectors, 12 μg of psPAX (Addgene; expressing gag-pol, packaging plasmid) and 2.4 μg of pMD.G plasmid (Addgene; expressing VSVG, envelope plasmid). In order to facilitate the transformation, lipofectamine (Invitrogen, USA) and PLUS reagent (Invitrogen, USA) were used. 6 hours after the transformation, the medium was replaced with DMEM supplemented with 10% fetal bovine serum. After 48 hours of additional culturing, the supernatant was collected.

The obtained supernatant was mixed with a lentivirus concentration kit (Lenti-X concentrator, Clontech Laboratories, USA) and then cultured overnight at 4°C. It was centrifuged under the condition of 4°C and 4,000 rpm for 2 hours to obtain viruses, which were then resuspended in 0.5 ml of DMEM not containing FBS. As a result, lentiviruses produced from pBD-1, pBD-2 and pBD-3 lentiviral vectors were prepared at the concentrations of $4.0 \times 10^8$ TU/ml, $2.0 \times 10^8$ TU/ml and $1.2 \times 10^9$ TU/ml, respectively.

### 1-3. Preparation of immortalized mesenchymal stem cells

Immortalized MSCs were prepared using the lentiviruses containing the immortalized genes produced in Example 1-2.

First, bone marrow-derived MSCs were prepared by the following method. Specifically, bone marrow aspirate was obtained from the iliac crest of a healthy donor. The aspirate was mixed with 20 IU/ml heparin in a sterile container to inhibit coagulation. The bone marrow mixture solution was centrifuged under the condition of 4°C, 739 g for 7 minutes, and then the supernatant was removed and the resultant was mixed with 10-fold amount (in volume) of sterilized water. The resultant mixture was centrifuged again under the same condition to obtain cell pellets. The obtained pellets were suspended in DMEM-low glucose (11885-084, Gibco, USA) supplemented with 20% FBS and 5 ng/ml b-FGF (100-18B, Peprotech, USA), which were then dispensed into a culture flask. It was cultured under the condition of 37°C, 5% $CO_2$ for 24 to 48 hours, and then replaced with a new medium. The cells were cultured with passages while the medium was replaced with new one at the interval of 3 to 4 days. After 2 weeks of culturing, MSCs were confirmed using a fluorescent cell analyzer.

The MSCs prepared above were infected with the pBD-1 lentiviruses produced in Example 1-2 at 100 MOI using Retronectin (Clontech Laboratories, USA). The infected cells were infected with the pBD-2 lentiviral vector at 100 MOI by the same method. After infection, the cells infected with pBD-2 lentiviruses were selected by adding 500 μg/ml zeomycin to the culture medium of the stabilized cells.

The selected cells were infected with pBD-3 lentiviral vector at 100 MOI. After infection, the cells infected with pBD-3 lentiviruses were selected by adding 1 μg/ml puromycin to the culture medium of the stabilized cells.

As a result, the cell proliferation rates of the MSCs containing the immortalized gene and the MSCs not containing the immortalized gene are shown in Fig. 1. As shown in Fig. 1, the MSCs infected with lentiviruses containing the immortalized genes, c-myc and hTERT, maintained high cell proliferation rates even after 120 days of culture. On the other hand, the cell proliferation rate of non-immortalized MSCs(MSC) decreased rapidly after 40 days of culture.

## Example 2. Construction of lentivirus containing TRAIL and CD genes

### 2-1. Construction of lentiviral vector containing TRAIL and CD genes

The TRAIL gene (SEQ ID NO: 2) and the CD gene (SEQ ID NO: 4) were inserted into the prepared pBD lentiviral vector. Herein, the inserted TRAIL and CD genes were linked via IRES (internal ribosome entry site), and the expression was designed to be regulated by the TRE promoter. IRES is a ribosome binding site that allows translation to begin without the 5'-cap structure, enabling the expression of two proteins by a single mRNA. On the other hand, the TRE promoter can regulate the expression of the gene linked to the promoter depending on the presence or absence of the addition of doxycycline.

The constructed vector was designated as pBD-4, and the structure of the gene construct is shown in Fig. 2.

### 2-2. Production of lentivirus containing TRAIL and CD genes

Using the lentiviral vector containing the TRAIL and CD genes constructed in Example 2-1, lentivirus was produced by the same method as described in Example 1-2. The lentivirus produced was prepared at a concentration of $7.6 \times 10^8$ TU/ml.

**Example 3. Preparation of MSC infected with lentivirus containing TRAIL and CD genes**

**3-1. Preparation of MSC infected with lentivirus containing TRAIL and CD genes**

Cells expressing TRAIL and CD genes were prepared by infecting the immortalized MSCs prepared in Example 1-3 with lentivirus containing TRAIL and CD genes produced in Example 2-2. The infection was carried out by the same method as described in Examples 1-3. After the infection, 1 μg/ml of doxycycline (631311, Clontech, USA) was added to the culture medium of the stabilized cells, and the cells were cultured in a state in which the expressions of TRAIL and CD were suppressed. After the cells were stabilized, TRAIL and CD expressions were induced by culturing the cells in cell media for 72 hours from which doxycycline was removed. Then the cells expressing TRAIL on the surface are isolated using FACS.

Specifically, the cells infected with lentivirus containing TRAIL and CD genes were dispensed into FACS tubes at $5 \times 10^5$ cells/tube, and centrifuged under the condition of 4°C and 1,500 rpm for 5 minutes, and the resulting supernatant was removed. The cells were resuspended by adding 1 ml of FACS buffer (PBS containing 2% fetal bovine serum) thereto, and centrifuged under the same condition and the resulting supernatant was removed. After the above washing procedure was conducted once more, the cells were resuspended in 1 ml of FACS buffer. The resuspended cells were added with a mixture prepared by adding 0.3 μl of LIVE/DEAD® Fixable Near-IR Dead Cell Stain (Life Technologies-Molecular Probes, USA) and and 5 μl of APC anti-human CD253 antibody (BioLegend, Cat #. 308210, USA), an anti-TRAIL antibody, to 200 μl of FACS buffer, and the resultant mixture was reacted at 4°C for 30 minutes. After the reaction, the cells were washed twice by the same method as above and the resulting supernatant was removed. 300 μl of fixing buffer (PBS containing 2% formaldehyde and 1% fetal bovine serum) was added to the washed cells and left at 4°C for at least 15 minutes. The cells were analyzed by the FACS (LSRFortessa, BD biosciences, USA) device and cells expressing TRAIL were isolated.

The isolated cells were cultured to form colonies. The monoclonal cells obtained from the colonies formed were cultured to establish a cell line, which was designated as BM-03. The cell line BM-03 was deposited on Jan. 6, 2017 with the deposit number KCTC 13182BP at Korean Collection for Type Cultures at Korea Research Institute of Bioscience & Biotechnology (KRIBB).

**3-2. Examination of surface antigen protein expression in established cell lines**

Surface antigen protein expression of bone marrow-derived MSCs before gene insertion and that of BM-03 cell line to which TRAIL and CD genes were inserted were analyzed using a human MSC assay kit (Stemflow™, Cat No 562245, BD). Experiments were conducted according to the manual included in each kit, and the experimental results are shown in Fig. 3.

As shown in Fig. 3, it was verified that the expressions of surface antigen proteins CD90, CD44, CD105, and CD73, inherent characteristics of MSCs, in BM-03 cell line which went through the gene manipulation to express TRAIL and CD therapeutic genes were the same as those in bone marrow-derived MSCs.

**3-3. Examination of differentiation ability of BM-03 cell line**

BM-03 cell line was seeded in a 12-well plate at a concentration of 1 x $10^4$ cells/$cm^2$ to examine adipogenesis. Using general culture medium, the cells were cultured in an incubator under the condition of 5% $CO_2$ and 37°C for 2 to 3 days, and then replaced with a adipogenesis differentiation medium (StemPro® adipogenesis differentiation kit, Thermo fisher Scientific, A10070-01). The cells were cultured for 21 days while the medium was changed every 3 or 4 days. When the culture was completed, the cells were stained with Oil Red O solution and examined with a microscope.

To examine osteogenesis, cells were seeded in a 12-well plate at a concentration of 5 x $10^3$ cells/$cm^2$. Using general culture medium, the cells were cultured in an incubator under the condition of 5% $CO_2$ and 37°C for 2 to 3 days, and then replaced with an osteogenesis differentiation medium (StemPro® osteogenesis differentiation kit, Thermo fisher Scientific, A10072-01). The cells were cultured for 21 days while the medium was changed every 3 or 4 days. When the culture was completed, the cells were stained with Alizarin Red S and examined with a microscope.

To examine chondrogenesis, cells were suspended at a concentration of 1.6 x $10^7$ cells/ml, and 5 μl of the suspension was seeded in a 24-well plate and cultured for 2 hours. 500 μl of chondrogenesis differentiation medium (StemPro® chondrogenesis differentiation kit, Thermo fisher Scientific, A 10071-01) was added thereto, and the cells were cultured for 14 days while the medium was changed every 3 days. After the culture, the medium was aspirated and the cells were rinsed with DPBS to take out pellets. After cryosection, the cells were stained with Alcian blue and examined by a microscope. The results are shown in Fig.4.

As shown in Fig. 4, it was verified that BM-03 cell line which went through the gene manipulation to express TRAIL and CD therapeutic genes had differentiation ability which is an inherent characteristic of MSCs.

### 3-4. Test for examination of gene introduced into BM-03 cell line

A sample of the established cell line, BM-03, was thawed for about 1 minute in a constant temperature water bath at 37°C, transferred to a 15 ml tube containing 9 ml PBS, and subjected to a Cell Down process for 5 minutes at 1,500 rpm. After PBS was completely removed, the pellet was suspended in 200 μl of PBS in a 1.5 ml tube, and transferred. gDNA was prepared using NucleoSpin® Tissue (MN, 740952.250), and the mixture was prepared as shown in Table 1 below, followed by PCR by the steps shown in Table 2 below. Herein, 100 ng of BM-03 plasmid DNA was added as a positive control and 1 μl of dW was used as a negative control.

[Table 1]

| | |
|---|---|
| Forward primer (SEQ ID NO: 17) (10 pmol/μl, GX535) | 1 μl |
| Reverse primer (SEQ ID NO: 18) (10 pmol/μl, GX534) | 1 μl |
| Sample (100 ng/μl) | 1 μl |
| dW | 17 μl |
| Total volume | 20 μl |

[Table 2]

| Step | Temperature | Time | Cycle |
|---|---|---|---|
| 1st | 95°C | 5 min | 1 |
| 2nd | 95°C | 45 sec | 35 |
| | 58°C | 45 sec | |
| | 72°C | 1 min | |
| 3rd | 72°C | 7 min | 1 |
| 4th | 4°C | Indefinitely | 1 |

1% agarose gel was placed in an electrophoresis kit. 10 μl of DNA Size Marker was loaded in the first well and 10 μl each of a negative control, a positive control, and BM-03 sample (x 3) were loaded in the following wells respectively in the above order. Thereafter, electrophoresis was conducted at 100 V for 20 minutes, and a gel photograph was taken. The result is shown in Fig. 5.

As shown in Fig. 5, all three of the BM-03 cell line samples showed PCR products of the same size (1.2 kb) as the positive control.

### 3-5. Identification of expression of TRAIL and CD proteins in established cell lines

In the BM-03 cell line established in Example 3-1, the expression of TRAIL and CD proteins were induced by the same method as in Example 3-1, and FACS was conducted to identify the expression of TRAIL according to the presence or absence of doxycycline. Then, the expression of CD protein was identified in the cell line in which the expression of TRAIL was identified.

Specifically, the BM-03 cell line was seeded in T75 flasks at a number of $5 \times 10^5$ cells using DMEM supplemented with 10% FBS containing 2 μg/ml of doxycycline, and DMEM supplemented with 10% FBS not containing 2 μg/ml of doxycycline. Cells were recovered after 3 days of culture. After measuring the number of cells, $5 \times 10^5$ cells per group were stained with PE anti-human CD253 (TRAIL) antibody (BioLegend, 308206) and PE Mouse IgG1, κ Isotype Control Antibody (BioLegend, 400112). In addition, to identify the expression after excluding dead cells, LIVE/DEAD® Fixable Near-IR Dead Cell Stain Kit (Thermos Fisher Scientific L34976) antibody was used. Samples were analyzed using FACS (LSRFortessa, BD Biosciences) device. After the cells were cultured by the same method, the expression of CD was induced by removing doxycycline. 48 hours after adding 5-FC (5-fluorocytosine, Sigma) thereto at a concentration of 100 μg/ml, apoptosis was observed. The results are shown in Fig. 6 and Fig. 7.

As shown in Fig. 6, it was found that TRAIL was not expressed when doxycycline was added, but TRAIL was expressed in the BM-03 cell line cultured in the medium from which the doxycycline was removed. Further, it was found that, as shown in Fig. 7, if the CD protein was expressed, cells were killed by 5FC. That is, the cells die owing to the expression of the CD protein. Accordingly, it was found that the TRAIL protein and CD protein were expressed in the prepared mesenchymal stem cells, which was regulated by the Tet-off system.

### 3-6. Cell population doubling level (PDL) analysis

The BM-03 cell line was seeded in a T75 flask at 4 x $10^5$ cells using a medium containing 2 μg/ml of doxycycline. Cells were obtained through subculture for about 3 or 4 days, and the total number of cells was counted. Cells of the same number were seeded, and PDL was measured every 3 or 4 days. The PDL was calculated using the following Equation 1, and the result is shown in Fig. 8. In the Equation 1, X represents the initial PDL, I represents the initial number of cells seeded in the blood vessel, Y represents the final cell yield or the number of cells in the end of growth period.

[Equation 1]

$$PDL = X + 3.222 (\log Y - \log I)$$

As shown in Fig. 8, stable growth was observed in long-term subculture.

### 3-7. Karyotype analysis of cells

In order to examine the chromosomal abnormality of the cells to which genes were introduced into the BM-03 cell line, analysis was requested to EONE Life Science Institute (Korea) and conducted in accordance with the protocol. The result of the analysis is shown in Fig. 9.

As shown in Fig. 9, no abnormality was observed in the chromosome of the BM-03 cells into which genes were introduced, and thus it was determined as a normal karyotype.

[Certificate of Microorganism Deposit]

BUDAPEST TREATY ON THE INTERNATIONAL RECOGNITION OF THE DEPOSIT OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: SL-BIGEN

SL-BIGEN
7th Fl. Bldg. C Korea Bio-Park, 700, Daewangpangyo-ro, Bundang-gu, Seongnam-si, Gyeonggi-do 13488
Republic of Korea

**I. IDENTIFICATION OF THE MICROORGANISM**

| Identification reference given by the DEPOSITOR: | Accession number given by the INTERNATIONAL DEPOSITARY AUTHORITY: |
| --- | --- |
| **BM-03** | **KCTC 13182BP** |

**II. SCIENTIFIC DESCRIPTION AND/OR PROPOSED TAXONOMIC DESIGNATION**

The microorganism identified under I above was accompanied by:

[ ] a scientific description

[ ] a proposed taxonomic designation

(Mark with a cross where applicable)

**III. RECEIPT AND ACCEPTANCE**

This International Depositary Authority accepts the microorganism identified under I above, which was received by it on January 06, 2017.

**IV. RECEIPT OF REQUEST FOR CONVERSION**

The microorganism identified under I above was received by this International Depositary Authority on and a request to convert the original deposit to a deposit under the Budapest Treaty was received by it on

**V. INTERNATIONAL DEPOSITARY AUTHORITY**

Name: **Korean Collection for Type Cultures**

Address: Korea Research Institute of Bioscience and Biotechnology (KRIBB)
181, Ipsin-gil, Jeongeup-si, Jeollabuk-do 56212
Republic of Korea

Signature(s) of person(s) having the power to represent the International Depositary Authority or of authorized official(s):

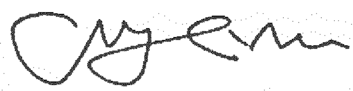

KIM, Cha Young, Director
Date: January 17, 2017

Form BP/4 (KCTC Form 17) sole page

<110> SLBIGEN Inc.

<120> MESENCHYMAL STEM CELL EXPRESSING TRAIL AND CD, AND USE THEREOF

<130> PCB701008SLB

<150> KR 2016-0015103
<151> 2016-02-05

<160> 18

<170> KoPatentIn 3.0

<210> 1
<211> 281
<212> PRT
<213> Artificial Sequence

<220>
<223> amino acid sequence of TRAIL

<400> 1
```
Met Ala Met Met Glu Val Gln Gly Gly Pro Ser Leu Gly Gln Thr Cys
  1               5                  10                  15

Val Leu Ile Val Ile Phe Thr Val Leu Leu Gln Ser Leu Cys Val Ala
             20                  25                  30

Val Thr Tyr Val Tyr Phe Thr Asn Glu Leu Lys Gln Met Gln Asp Lys
         35                  40                  45

Tyr Ser Lys Ser Gly Ile Ala Cys Phe Leu Lys Glu Asp Asp Ser Tyr
     50                  55                  60

Trp Asp Pro Asn Asp Glu Glu Ser Met Asn Ser Pro Cys Trp Gln Val
 65                  70                  75                  80

Lys Trp Gln Leu Arg Gln Leu Val Arg Lys Met Ile Leu Arg Thr Ser
                 85                  90                  95

Glu Glu Thr Ile Ser Thr Val Gln Glu Lys Gln Gln Asn Ile Ser Pro
            100                 105                 110

Leu Val Arg Glu Arg Gly Pro Gln Arg Val Ala Ala His Ile Thr Gly
        115                 120                 125

Thr Arg Gly Arg Ser Asn Thr Leu Ser Ser Pro Asn Ser Lys Asn Glu
    130                 135                 140

Lys Ala Leu Gly Arg Lys Ile Asn Ser Trp Glu Ser Ser Arg Ser Gly
145                 150                 155                 160

His Ser Phe Leu Ser Asn Leu His Leu Arg Asn Gly Glu Leu Val Ile
                165                 170                 175

His Glu Lys Gly Phe Tyr Tyr Ile Tyr Ser Gln Thr Tyr Phe Arg Phe
            180                 185                 190

Gln Glu Glu Ile Lys Glu Asn Thr Lys Asn Asp Lys Gln Met Val Gln
        195                 200                 205

Tyr Ile Tyr Lys Tyr Thr Ser Tyr Pro Asp Pro Ile Leu Leu Met Lys
```

210 215 220

Ser Ala Arg Asn Ser Cys Trp Ser Lys Asp Ala Glu Tyr Gly Leu Tyr
225 230 235 240

Ser Ile Tyr Gln Gly Gly Ile Phe Glu Leu Lys Glu Asn Asp Arg Ile
245 250 255

Phe Val Ser Val Thr Asn Glu His Leu Ile Asp Met Asp His Glu Ala
260 265 270

Ser Phe Phe Gly Ala Phe Leu Val Gly
275 280

<210> 2
<211> 846
<212> DNA
<213> Artificial Sequence

<220>
<223> nucleotide sequence of TRAIL

<400> 2
atggcaatga tggaggtcca gggggggcct tcactgggac agacatgcgt gctgatcgtc 60

atctttaccg tgctgctgca gtcactgtgc gtggctgtca catacgtgta tttcactaac 120

gagctgaagc agatgcagga caagtactca aaaagcggaa tcgcatgctt tctgaaagaa 180

gacgatagct attgggaccc taacgatgag gaatccatga actccccatg ttggcaggtg 240

aagtggcagc tgcgacagct ggtccggaaa atgatcctga ggactagtga ggaaactatt 300

tcaaccgtgc aggagaagca gcagaatatc agcccactgg tgcgggaaag aggaccacag 360

cgagtcgcag ctcacattac cggaacaagg ggccgcagca acaccctgag ctccccaaac 420

tccaagaatg agaaagccct gggcagaaag atcaattcct gggaatctag taggagtggg 480

cactcattcc tgagcaacct gcatctgcgc aatggggagc tggtcatcca tgaaaaaggc 540

ttctactata tctactctca gacctatttc cgatttcagg aggaaattaa ggagaacaca 600

aagaatgaca aacagatggt ccagtacatc tataaataca catcttaccc cgatcctatt 660

ctgctgatga agagtgcacg gaactcctgt tggtctaaag acgccgagta tgggctgtac 720

agcatctatc agggcgggat tttcgagctg aaggaaaatg atagaatctt tgtgagcgtc 780

accaacgagc atctgattga catggatcac gaggcttcat tttcggggc attcctggtc 840

ggataa 846

<210> 3
<211> 373
<212> PRT
<213> Artificial Sequence

<220>
<223> amino acid sequence of CD::UPRT

<400> 3

```
Met Val Thr Gly Gly Met Ala Ser Lys Trp Asp Gln Lys Gly Met Asp
 1               5                  10                  15

Ile Ala Tyr Glu Glu Ala Ala Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Val Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Ile Met Lys Gln Phe
    130                 135                 140

Ile Asp Glu Arg Pro Gln Asp Trp Phe Glu Asp Ile Gly Glu Ser Ser
145                 150                 155                 160

Glu Pro Phe Lys Asn Val Tyr Leu Leu Pro Gln Thr Asn Gln Leu Leu
                165                 170                 175

Gly Leu Tyr Thr Ile Ile Arg Asn Lys Asn Thr Thr Arg Pro Asp Phe
            180                 185                 190

Ile Phe Tyr Ser Asp Arg Ile Ile Arg Leu Leu Val Glu Glu Gly Leu
        195                 200                 205

Asn His Leu Pro Val Gln Lys Gln Ile Val Glu Thr Asp Thr Asn Glu
    210                 215                 220

Asn Phe Glu Gly Val Ser Phe Met Gly Lys Ile Cys Gly Val Ser Ile
225                 230                 235                 240

Val Arg Ala Gly Glu Ser Met Glu Gln Gly Leu Arg Asp Cys Cys Arg
                245                 250                 255

Ser Val Arg Ile Gly Lys Ile Leu Ile Gln Arg Asp Glu Glu Thr Ala
            260                 265                 270

Leu Pro Lys Leu Phe Tyr Glu Lys Leu Pro Glu Asp Ile Ser Glu Arg
        275                 280                 285

Tyr Val Phe Leu Leu Asp Pro Met Leu Ala Thr Gly Gly Ser Ala Ile
    290                 295                 300

Met Ala Thr Glu Val Leu Ile Lys Arg Gly Val Lys Pro Glu Arg Ile
305                 310                 315                 320
```

Tyr Phe Leu Asn Leu Ile Cys Ser Lys Glu Gly Ile Glu Lys Tyr His
325 330 335

Ala Ala Phe Pro Glu Val Arg Ile Val Thr Gly Ala Leu Asp Arg Gly
340 345 350

Leu Asp Glu Asn Lys Tyr Leu Val Pro Gly Leu Gly Asp Phe Gly Asp
355 360 365

Arg Tyr Tyr Cys Val
370

<210> 4
<211> 1122
<212> DNA
<213> Artificial Sequence

<220>
<223> nucleotide sequence of CD::UPRT

<400> 4
atggtgaccg gcggcatggc cagcaagtgg gaccagaagg gcatggacat cgcctacgag 60

gaggccgctc tgggctacaa ggagggcggc gtgcccatcg gcggatgcct gatcaacaac 120

aaggacggca gcgtgctggg cagaggccac aacatgagat tccagaaggg cagcgccacc 180

ctgcacggcg agatcagcac cctggagaac tgcggcagac tggagggcaa ggtgtacaag 240

gacacaaccc tgtacaccac actgagcccc tgcgacatgt gcaccggcgc catcatcatg 300

tacggcatcc ccagatgcgt ggtgggcgag aacgtgaact tcaagagcaa gggcgagaag 360

tacctgcaga ccagaggcca cgaggtggtc gtcgtggacg acgagagatg caagaagatc 420

atgaagcagt tcatcgacga gagaccccag gactggttcg aggacatcgg cgagagcagc 480

gagcccttca agaacgtgta cctgctgccc cagaccaacc agctgctggg cctgtacacc 540

atcatcagaa acaagaacac caccagaccc gacttcatct tctacagcga cagaatcatc 600

agactgctgg tggaggaggg cctgaaccac ctgcccgtgc agaagcagat cgtggagacc 660

gacaccaacg agaacttcga gggcgtgagc ttcatgggca aaatctgcgg cgtgagcatc 720

gtgagagccg gcgagagcat ggagcagggc ctgagagact gctgcagaag cgtgagaatc 780

ggcaagatcc tgatccagag agacgaggag accgccctgc ccaagctgtt ctacgagaag 840

ctgcccgagg acatcagcga gagatacgtg ttcctgctgg accccatgct ggccaccggc 900

ggcagcgcca tcatggccac cgaggtgctg atcaagagag gcgtgaagcc cgagagaatc 960

tacttcctga acctgatctg cagcaaggag ggcatcgaga agtaccacgc tgccttcccc 1020

gaggtgagaa tcgtgaccgg cgccctggac agaggcctgg acgagaacaa gtacctggtg 1080

cccggcctgg gcgacttcgg cgacagatac tactgcgtgt aa 1122

<210> 5

<211> 376
<212> PRT
<213> Artificial Sequence

<220>
<223> amino acid sequence of TK

<400> 5
```
Met Ala Ser Tyr Pro Cys His Gln His Ala Ser Ala Phe Asp Gln Ala
1               5                   10                  15

Ala Arg Ser Arg Gly His Ser Asn Arg Arg Thr Ala Leu Arg Pro Arg
            20                  25                  30

Arg Gln Gln Glu Ala Thr Glu Val Arg Leu Glu Gln Lys Met Pro Thr
        35                  40                  45

Leu Leu Arg Val Tyr Ile Asp Gly Pro His Gly Met Gly Lys Thr Thr
    50                  55                  60

Thr Thr Gln Leu Leu Val Ala Leu Gly Ser Arg Asp Asp Ile Val Tyr
65                  70                  75                  80

Val Pro Glu Pro Met Thr Tyr Trp Gln Val Leu Gly Ala Ser Glu Thr
                85                  90                  95

Ile Ala Asn Ile Tyr Thr Thr Gln His Arg Leu Asp Gln Gly Glu Ile
            100                 105                 110

Ser Ala Gly Asp Ala Ala Val Val Met Thr Ser Ala Gln Ile Thr Met
        115                 120                 125

Gly Met Pro Tyr Ala Val Thr Asp Ala Val Leu Ala Pro His Val Gly
    130                 135                 140

Gly Glu Ala Gly Ser Ser His Ala Pro Pro Pro Ala Leu Thr Leu Ile
145                 150                 155                 160

Phe Asp Arg His Pro Ile Ala Ala Leu Leu Cys Tyr Pro Ala Ala Arg
                165                 170                 175

Tyr Leu Met Gly Ser Met Thr Pro Gln Ala Val Leu Ala Phe Val Ala
            180                 185                 190

Leu Ile Pro Pro Thr Leu Pro Gly Thr Asn Ile Val Leu Gly Ala Leu
        195                 200                 205

Pro Glu Asp Arg His Ile Asp Arg Leu Ala Lys Arg Gln Arg Pro Gly
    210                 215                 220

Glu Arg Leu Asp Leu Ala Met Leu Ala Ala Ile Arg Arg Val Tyr Gly
225                 230                 235                 240

Leu Leu Ala Asn Thr Val Arg Tyr Leu Gln Gly Gly Gly Ser Trp Trp
                245                 250                 255

Glu Asp Trp Gly Gln Leu Ser Gly Thr Ala Val Pro Pro Gln Gly Ala
            260                 265                 270

Glu Pro Gln Ser Asn Ala Gly Pro Arg Pro His Ile Gly Asp Thr Leu
        275                 280                 285
```

```
Phe Thr Leu Phe Arg Ala Pro Glu Leu Leu Ala Pro Asn Gly Asp Leu
    290                 295                 300

Tyr Asn Val Phe Ala Trp Ala Leu Asp Val Leu Ala Lys Arg Leu Arg
305                 310                 315                 320

Pro Met His Val Phe Ile Leu Asp Tyr Asp Gln Ser Pro Ala Gly Cys
                325                 330                 335

Arg Asp Ala Leu Leu Gln Leu Thr Ser Gly Met Val Gln Thr His Val
            340                 345                 350

Thr Thr Pro Gly Ser Ile Pro Thr Ile Cys Asp Leu Ala Arg Thr Phe
        355                 360                 365

Ala Arg Glu Met Gly Glu Ala Asn
    370                 375


<210>  6
<211>  1131
<212>  DNA
<213>  Artificial Sequence

<220>
<223>  nucleotide sequence of TK


<400>  6
atggccagct acccctgtca ccagcacgcc agcgccttcg accaggccgc tagaagcaga        60

ggccacagca acagaagaac cgccctgaga cccagaagac agcaggaggc cacagaggtg       120

agactggagc agaagatgcc caccctgctg agagtgtaca tcgatggacc ccacggcatg       180

ggcaagacca caacaaccca gctgctggtg gccctgggca gcagagacga catcgtgtac       240

gtgcccgagc ccatgaccta ctggcaggtg ctgggagcca gcgagaccat cgccaacatc       300

tacaccacac agcacagact ggaccagggc gagatcagcg ccggcgacgc tgccgtggtg       360

atgaccagcg cccagatcac aatgggcatg ccctacgccg tgaccgatgc cgtgctggct       420

ccccacgtgg gcggagaggc cggcagcagc cacgcccctc cccctgccct gaccctgatc       480

ttcgacagac accccatcgc cgccctgctg tgctaccccg ccgctagata cctgatgggc       540

agcatgacac cccaggccgt gctggccttc gtggccctga tcccccctac cctgcccggc       600

accaacatcg tgctgggcgc cctgcccgag gacagacaca tcgacagact ggctaagaga       660

cagagacccg gcgagagact ggacctggcc atgctggccg ccatcagaag agtgtacggc       720

ctgctggcca acaccgtgag atacctgcag ggaggcggca gctggtggga ggactggggc       780

cagctgagcg gcaccgccgt gcctccccag ggcgccgagc cccagagcaa cgccggccct       840

agaccccaca tcggcgacac cctgttcacc ctgtttagag cccccgagct gctggccccc       900

aacggcgacc tgtacaacgt gttcgcctgg gccctggacg tgctggccaa gagactgaga       960

cccatgcacg tgttcatcct ggactacgac cagagccccg ccggctgcag agatgccctg      1020
```

```
ctgcagctga ccagcggcat ggtgcagacc cacgtgacca cacccggcag catccccacc       1080

atctgcgacc tggccagaac ctttgccaga gagatgggcg aggccaactg a                1131


<210>    7
<211>    454
<212>    PRT
<213>    Artificial Sequence

<220>
<223>    amino acid sequence of c-myc


<400>    7
Met Asp Phe Phe Arg Val Val Glu Asn Gln Gln Pro Pro Ala Thr Met
1               5                   10                  15

Pro Leu Asn Val Ser Phe Thr Asn Arg Asn Tyr Asp Leu Asp Tyr Asp
            20                  25                  30

Ser Val Gln Pro Tyr Phe Tyr Cys Asp Glu Glu Glu Asn Phe Tyr Gln
        35                  40                  45

Gln Gln Gln Gln Ser Glu Leu Gln Pro Pro Ala Pro Ser Glu Asp Ile
    50                  55                  60

Trp Lys Lys Phe Glu Leu Leu Pro Thr Pro Pro Leu Ser Pro Ser Arg
65                  70                  75                  80

Arg Ser Gly Leu Cys Ser Pro Ser Tyr Val Ala Val Thr Pro Phe Ser
                85                  90                  95

Leu Arg Gly Asp Asn Asp Gly Gly Gly Gly Ser Phe Ser Thr Ala Asp
            100                 105                 110

Gln Leu Glu Met Val Thr Glu Leu Leu Gly Gly Asp Met Val Asn Gln
        115                 120                 125

Ser Phe Ile Cys Asp Pro Asp Asp Glu Thr Phe Ile Lys Asn Ile Ile
    130                 135                 140

Ile Gln Asp Cys Met Trp Ser Gly Phe Ser Ala Ala Ala Lys Leu Val
145                 150                 155                 160

Ser Glu Lys Leu Ala Ser Tyr Gln Ala Ala Arg Lys Asp Ser Gly Ser
                165                 170                 175

Pro Asn Pro Ala Arg Gly His Ser Val Cys Ser Thr Ser Ser Leu Tyr
            180                 185                 190

Leu Gln Asp Leu Ser Ala Ala Ala Ser Glu Cys Ile Asp Pro Ser Val
        195                 200                 205

Val Phe Pro Tyr Pro Leu Asn Asp Ser Ser Ser Pro Lys Ser Cys Ala
    210                 215                 220

Ser Gln Asp Ser Ser Ala Phe Ser Pro Ser Ser Asp Ser Leu Leu Ser
225                 230                 235                 240

Ser Thr Glu Ser Ser Pro Gln Gly Ser Pro Glu Pro Leu Val Leu His
                245                 250                 255
```

```
Glu Glu Thr Pro Pro Thr Thr Ser Ser Asp Ser Glu Glu Glu Gln Glu
            260                 265                 270

Asp Glu Glu Glu Ile Asp Val Val Ser Val Glu Lys Arg Gln Ala Pro
        275                 280                 285

Gly Lys Arg Ser Glu Ser Gly Ser Pro Ser Ala Gly Gly His Ser Lys
    290                 295                 300

Pro Pro His Ser Pro Leu Val Leu Lys Arg Cys His Val Ser Thr His
305                 310                 315                 320

Gln His Asn Tyr Ala Ala Pro Pro Ser Thr Arg Lys Asp Tyr Pro Ala
                325                 330                 335

Ala Lys Arg Val Lys Leu Asp Ser Val Arg Val Leu Arg Gln Ile Ser
            340                 345                 350

Asn Asn Arg Lys Cys Thr Ser Pro Arg Ser Ser Asp Thr Glu Glu Asn
        355                 360                 365

Val Lys Arg Arg Thr His Asn Val Leu Glu Arg Gln Arg Arg Asn Glu
    370                 375                 380

Leu Lys Arg Ser Phe Phe Ala Leu Arg Asp Gln Ile Pro Glu Leu Glu
385                 390                 395                 400

Asn Asn Glu Lys Ala Pro Lys Val Val Ile Leu Lys Lys Ala Thr Ala
                405                 410                 415

Tyr Ile Leu Ser Val Gln Ala Glu Glu Gln Lys Leu Ile Ser Glu Glu
            420                 425                 430

Asp Leu Leu Arg Lys Arg Arg Glu Gln Leu Lys His Lys Leu Glu Gln
        435                 440                 445

Leu Arg Asn Ser Cys Ala
    450


<210>   8
<211>   1365
<212>   DNA
<213>   Artificial Sequence

<220>
<223>   nucleotide sequence of c-myc


<400>   8
atggatttct ttcgcgtcgt cgagaaccag cagccacccg ccactatgcc tctgaacgtg      60

tcttttacta acaggaacta tgatctggat tacgacagcg tgcagcccta cttctattgc     120

gatgaggaag agaactttta tcagcagcag cagcagagcg agctgcagcc acctgcacct     180

tccgaagaca tttggaagaa attcgagctg ctgcctacac cacccctgtc tccaagtcgg     240

agaagcggcc tgtgttcacc cagctacgtg gccgtcactc ctttcagcct gagggggac     300

aatgatggcg ggggaggctc cttttctaca gccgatcagc tggaaatggt gactgagctg     360

ctgggggggag acatggtcaa ccagagcttc atttgcgatc ctgacgatga aacttttatc     420
```

aagaacatca tcatccagga ctgtatgtgg tcaggcttta gcgccgctgc aaagctggtg 480

tctgagaaac tggcaagtta tcaggccgct cggaaagata gtgggtcacc taacccagct 540

agaggacact ccgtgtgctc tacaagctcc ctgtacctgc aggacctgag cgcagccgct 600

tccgagtgta ttgatccctc cgtggtcttc cctatcctc tgaatgactc tagttcaccc 660

aagagttgcg catcacagga cagctccgcc ttttcacctt ctagtgatag cctgctgtca 720

agcactgaat cctctccaca gggcagccca gagccactgg tgctgcatga agagacccct 780

ccaaccacaa gttcagattc cgaagaggaa caggaggacg aggaagagat cgatgtggtc 840

tctgtggaaa agcgccaggc tccaggaaaa cgaagcgagt ccggctctcc aagtgcagga 900

ggacactcca agccacctca ttctcccctg gtgctgaaaa ggtgccacgt ctccacccac 960

cagcataact acgcagcccc accctctaca cgaaaggact atccagctgc aaagcgcgtg 1020

aaactggata gcgtgagagt cctgaggcag atcagtaaca atcggaagtg tacttcaccc 1080

agaagctccg acaccgaaga gaacgtgaaa aggcgcaccc ataatgtcct ggaacgccag 1140

cgacggaatg agctgaagag gtccttcttt gccctgcgcg atcagattcc tgaactggag 1200

aacaatgaga aggctccaaa agtggtcatt ctgaagaaag ccacagctta catcctgtct 1260

gtgcaggccg aagagcagaa actgatcagt gaagaggacc tgctgagaaa acgcagggaa 1320

cagctgaaac ataaactgga acagctgaga aactcttgtg cttaa 1365

<210> 9
<211> 1132
<212> PRT
<213> Artificial Sequence

<220>
<223> amino acid sequence of TERT

<400> 9

Met Pro Arg Ala Pro Arg Cys Arg Ala Val Arg Ser Leu Leu Arg Ser
1 5 10 15

His Tyr Arg Glu Val Leu Pro Leu Ala Thr Phe Val Arg Arg Leu Gly
20 25 30

Pro Gln Gly Trp Arg Leu Val Gln Arg Gly Asp Pro Ala Ala Phe Arg
35 40 45

Ala Leu Val Ala Gln Cys Leu Val Cys Val Pro Trp Asp Ala Arg Pro
50 55 60

Pro Pro Ala Ala Pro Ser Phe Arg Gln Val Ser Cys Leu Lys Glu Leu
65 70 75 80

Val Ala Arg Val Leu Gln Arg Leu Cys Glu Arg Gly Ala Lys Asn Val
85 90 95

Leu Ala Phe Gly Phe Ala Leu Leu Asp Gly Ala Arg Gly Gly Pro Pro

```
            100                 105                 110

Glu Ala Phe Thr Thr Ser Val Arg Ser Tyr Leu Pro Asn Thr Val Thr
        115                 120                 125

Asp Ala Leu Arg Gly Ser Gly Ala Trp Gly Leu Leu Leu Arg Arg Val
    130                 135                 140

Gly Asp Asp Val Leu Val His Leu Leu Ala Arg Cys Ala Leu Phe Val
145                 150                 155                 160

Leu Val Ala Pro Ser Cys Ala Tyr Gln Val Cys Gly Pro Pro Leu Tyr
                165                 170                 175

Gln Leu Gly Ala Ala Thr Gln Ala Arg Pro Pro Pro His Ala Ser Gly
            180                 185                 190

Pro Arg Arg Arg Leu Gly Cys Glu Arg Ala Trp Asn His Ser Val Arg
        195                 200                 205

Glu Ala Gly Val Pro Leu Gly Leu Pro Ala Pro Gly Ala Arg Arg Arg
    210                 215                 220

Gly Gly Ser Ala Ser Arg Ser Leu Pro Leu Pro Lys Arg Pro Arg Arg
225                 230                 235                 240

Gly Ala Ala Pro Glu Pro Glu Arg Thr Pro Val Gly Gln Gly Ser Trp
                245                 250                 255

Ala His Pro Gly Arg Thr Arg Gly Pro Ser Asp Arg Gly Phe Cys Val
            260                 265                 270

Val Ser Pro Ala Arg Pro Ala Glu Glu Ala Thr Ser Leu Glu Gly Ala
        275                 280                 285

Leu Ser Gly Thr Arg His Ser His Pro Ser Val Gly Arg Gln His His
    290                 295                 300

Ala Gly Pro Pro Ser Thr Ser Arg Pro Pro Arg Pro Trp Asp Thr Pro
305                 310                 315                 320

Cys Pro Pro Val Tyr Ala Glu Thr Lys His Phe Leu Tyr Ser Ser Gly
                325                 330                 335

Asp Lys Glu Gln Leu Arg Pro Ser Phe Leu Leu Ser Ser Leu Arg Pro
            340                 345                 350

Ser Leu Thr Gly Ala Arg Arg Leu Val Glu Thr Ile Phe Leu Gly Ser
        355                 360                 365

Arg Pro Trp Met Pro Gly Thr Pro Arg Arg Leu Pro Arg Leu Pro Gln
    370                 375                 380

Arg Tyr Trp Gln Met Arg Pro Leu Phe Leu Glu Leu Leu Gly Asn His
385                 390                 395                 400

Ala Gln Cys Pro Tyr Gly Val Leu Leu Lys Thr His Cys Pro Leu Arg
                405                 410                 415

Ala Ala Val Thr Pro Ala Ala Gly Val Cys Ala Arg Glu Lys Pro Gln
            420                 425                 430

Gly Ser Val Ala Ala Pro Glu Glu Glu Asp Thr Asp Pro Arg Arg Leu
```

435 440 445

Val Gln Leu Leu Arg Gln His Ser Ser Pro Trp Gln Val Tyr Gly Phe
450 455 460

Val Arg Ala Cys Leu Arg Arg Leu Val Pro Pro Gly Leu Trp Gly Ser
465 470 475 480

Arg His Asn Glu Arg Arg Phe Leu Arg Asn Thr Lys Lys Phe Ile Ser
485 490 495

Leu Gly Lys His Ala Lys Leu Ser Leu Gln Glu Leu Thr Trp Lys Met
500 505 510

Ser Val Arg Asp Cys Ala Trp Leu Arg Arg Ser Pro Gly Val Gly Cys
515 520 525

Val Pro Ala Ala Glu His Arg Leu Arg Glu Glu Ile Leu Ala Lys Phe
530 535 540

Leu His Trp Leu Met Ser Val Tyr Val Val Glu Leu Leu Arg Ser Phe
545 550 555 560

Phe Tyr Val Thr Glu Thr Thr Phe Gln Lys Asn Arg Leu Phe Phe Tyr
565 570 575

Arg Lys Ser Val Trp Ser Lys Leu Gln Ser Ile Gly Ile Arg Gln His
580 585 590

Leu Lys Arg Val Gln Leu Arg Glu Leu Ser Glu Ala Glu Val Arg Gln
595 600 605

His Arg Glu Ala Arg Pro Ala Leu Leu Thr Ser Arg Leu Arg Phe Ile
610 615 620

Pro Lys Pro Asp Gly Leu Arg Pro Ile Val Asn Met Asp Tyr Val Val
625 630 635 640

Gly Ala Arg Thr Phe Arg Arg Glu Lys Arg Ala Glu Arg Leu Thr Ser
645 650 655

Arg Val Lys Ala Leu Phe Ser Val Leu Asn Tyr Glu Arg Ala Arg Arg
660 665 670

Pro Gly Leu Leu Gly Ala Ser Val Leu Gly Leu Asp Asp Ile His Arg
675 680 685

Ala Trp Arg Thr Phe Val Leu Arg Val Arg Ala Gln Asp Pro Pro Pro
690 695 700

Glu Leu Tyr Phe Val Lys Val Asp Val Thr Gly Ala Tyr Asp Thr Ile
705 710 715 720

Pro Gln Asp Arg Leu Thr Glu Val Ile Ala Ser Ile Ile Lys Pro Gln
725 730 735

Asn Thr Tyr Cys Val Arg Arg Tyr Ala Val Val Gln Lys Ala Ala His
740 745 750

Gly His Val Arg Lys Ala Phe Lys Ser His Val Ser Thr Leu Thr Asp
755 760 765

Leu Gln Pro Tyr Met Arg Gln Phe Val Ala His Leu Gln Glu Thr Ser

770 775 780

Pro Leu Arg Asp Ala Val Val Ile Glu Gln Ser Ser Ser Leu Asn Glu
785 790 795 800

Ala Ser Ser Gly Leu Phe Asp Val Phe Leu Arg Phe Met Cys His His
805 810 815

Ala Val Arg Ile Arg Gly Lys Ser Tyr Val Gln Cys Gln Gly Ile Pro
820 825 830

Gln Gly Ser Ile Leu Ser Thr Leu Leu Cys Ser Leu Cys Tyr Gly Asp
835 840 845

Met Glu Asn Lys Leu Phe Ala Gly Ile Arg Arg Asp Gly Leu Leu Leu
850 855 860

Arg Leu Val Asp Asp Phe Leu Leu Val Thr Pro His Leu Thr His Ala
865 870 875 880

Lys Thr Phe Leu Arg Thr Leu Val Arg Gly Val Pro Glu Tyr Gly Cys
885 890 895

Val Val Asn Leu Arg Lys Thr Val Val Asn Phe Pro Val Glu Asp Glu
900 905 910

Ala Leu Gly Gly Thr Ala Phe Val Gln Met Pro Ala His Gly Leu Phe
915 920 925

Pro Trp Cys Gly Leu Leu Leu Asp Thr Arg Thr Leu Glu Val Gln Ser
930 935 940

Asp Tyr Ser Ser Tyr Ala Arg Thr Ser Ile Arg Ala Ser Leu Thr Phe
945 950 955 960

Asn Arg Gly Phe Lys Ala Gly Arg Asn Met Arg Arg Lys Leu Phe Gly
965 970 975

Val Leu Arg Leu Lys Cys His Ser Leu Phe Leu Asp Leu Gln Val Asn
980 985 990

Ser Leu Gln Thr Val Cys Thr Asn Ile Tyr Lys Ile Leu Leu Leu Gln
995 1000 1005

Ala Tyr Arg Phe His Ala Cys Val Leu Gln Leu Pro Phe His Gln Gln
1010 1015 1020

Val Trp Lys Asn Pro Thr Phe Phe Leu Arg Val Ile Ser Asp Thr Ala
1025 1030 1035 1040

Ser Leu Cys Tyr Ser Ile Leu Lys Ala Lys Asn Ala Gly Met Ser Leu
1045 1050 1055

Gly Ala Lys Gly Ala Ala Gly Pro Leu Pro Ser Glu Ala Val Gln Trp
1060 1065 1070

Leu Cys His Gln Ala Phe Leu Leu Lys Leu Thr Arg His Arg Val Thr
1075 1080 1085

Tyr Val Pro Leu Leu Gly Ser Leu Arg Thr Ala Gln Thr Gln Leu Ser
1090 1095 1100

Arg Lys Leu Pro Gly Thr Thr Leu Thr Ala Leu Glu Ala Ala Ala Asn

1105 1110 1115 1120

Pro Ala Leu Pro Ser Asp Phe Lys Thr Ile Leu Asp
1125 1130

<210> 10
<211> 3399
<212> DNA
<213> Artificial Sequence

<220>
<223> nucleotide sequence of hTERT

<400> 10
atgcccagag ctcccagatg cagagccgtg agaagcctgc tgagaagcca ctacagagag 60

gtgctgcccc tggccacctt cgtgagaaga ctgggacccc agggctggag actggtgcag 120

agaggcgacc ccgcagcctt tagagccctg gtggcccagt gcctggtgtg cgtgccctgg 180

gacgccagac ctcctcccgc tgcccccagc ttcagacagg tgagctgcct gaaggagctg 240

gtggccagag tgctccagag actgtgcgag agaggcgcca agaacgtgct ggcctttggc 300

ttcgccctgc tggatggagc cagaggcgga cctcccgagg ccttcaccac aagcgtgaga 360

agctacctgc ccaacaccgt gaccgatgcc ctgagaggct ccggcgcctg gggcctgctc 420

ctgagaagag tgggcgacga cgtgctggtg cacctgctgg ccagatgcgc cctgttcgtg 480

ctggtggctc ccagctgcgc ctaccaggtg tgcggacccc ctctgtacca gctgggagcc 540

gccacccagg caagaccccc tccccacgcc tctggaccca gaagaagact gggctgcgag 600

agagcctgga accacagcgt gagagaggct ggcgtgcccc tgggcctgcc cgcccctggc 660

gccagaagaa gaggcggcag cgccagcaga agcctgcccc tgcccaagag acccagacgc 720

ggcgccgctc ccgagcctga gagaacaccc gtgggccagg gcagctgggc ccaccccggc 780

agaaccagag gacccagcga cagaggcttc tgcgtggtga gccctgccag acccgccgag 840

gaggccacca gcctggaggg cgccctgagc ggcaccagac acagccaccc cagcgtgggc 900

agacagcacc acgccggccc tcctagcacc agcagacccc ccagaccttg ggacaccccc 960

tgcccccctg tgtacgccga gaccaagcac ttcctgtaca gcagcggcga caaggagcag 1020

ctgagaccca gcttcctgct gagctccctg agacccagcc tgaccggcgc cagaagactg 1080

gtggagacca tcttcctggg cagcagaccc tggatgcccg gcacccccag aagactgccc 1140

agactgcccc agagatactg gcagatgaga cccctgttcc tggagctgct gggcaaccac 1200

gcccagtgcc cctacggcgt gctgctgaag acccactgcc ccctgagagc tgccgtgacc 1260

cccgcagctg gcgtgtgcgc cagagagaag ccccagggca gcgtggccgc tcccgaggag 1320

gaggacaccg atcccagaag actggtgcag ctgctgagac agcacagcag cccctggcag 1380

gtgtacggct tcgtgagagc ctgcctgaga agactggtgc ctcccggcct gtggggcagc 1440

agacacaacg agagaagatt cctgagaaac accaagaagt tcatcagcct gggcaagcac 1500
gccaagctga gcctccagga gctgacatgg aagatgagcg tgagagactg cgcctggctg 1560
aggagaagcc ctggcgtggg ctgcgtgccc gccgccgagc acagactgag agaggagatc 1620
ctggccaagt ttctgcactg gctgatgagc gtgtacgtgg tggagctgct gagaagcttc 1680
ttctacgtga ccgagaccac attccagaag aacagactgt tcttttacag gaagagcgtg 1740
tggagcaagc tccagagcat cggcatcaga cagcacctga agagagtgca gctgagagag 1800
ctgagcgagg ccgaggtgag acagcacaga gaggccagac ccgccctgct gaccagcaga 1860
ctgagattca tccccaagcc cgatggcctg agacccatcg tgaacatgga ctacgtggtg 1920
ggagccagaa cctttagaag agagaagaga gccgagagac tgaccagcag agtgaaggcc 1980
ctgttcagcg tgctgaacta cgagagagcc agaagacccg gcctgctggg cgccagcgtg 2040
ctgggcctgg acgacatcca cagagcctgg agaaccttcg tgctgagagt gagagcccag 2100
gaccctcctc ccgagctgta cttcgtgaag gtggacgtga ccggcgccta cgacaccatc 2160
ccccaggaca gactgaccga ggtgatcgcc agcatcatca agccccagaa cacctactgc 2220
gtgagaagat acgccgtggt gcagaaggcc gcccacggcc acgtgagaaa ggccttcaag 2280
agccacgtga gcaccctgac cgacctccag ccctacatga gacagttcgt ggcccacctc 2340
caggagacca gcccccctgag agatgccgtg gtgatcgagc agagctcttc cctgaacgag 2400
gcctccagcg gcctgttcga cgtgttcctg agattcatgt gccaccacgc cgtgagaatc 2460
agaggcaaga gctacgtgca gtgccagggc atcccccagg gcagcatcct gagcaccctg 2520
ctgtgcagcc tgtgctacgg cgacatggag aacaagctgt tcgctggcat cagaagagac 2580
ggcctgctgc tgagactggt ggacgacttc ctgctggtga cccccccacct gacccacgcc 2640
aagaccttcc tgagaaccct ggtgagaggc gtgcccgagt acggctgcgt ggtgaacctg 2700
agaaagaccg tggtgaactt tcccgtggag gacgaggccc tgggcggcac cgccttcgtg 2760
cagatgcccg cccacggcct gtttccctgg tgcggcctgc tcctcgacac cagaaccctg 2820
gaggtgcaga gcgactacag cagctacgca agaaccagca tcagagccag cctgaccttc 2880
aacagaggct tcaaggccgg cagaaacatg agaagaaagc tgttcggcgt gctgagactg 2940
aagtgccaca gcctgttcct ggacctccag gtgaacagcc tccagaccgt gtgcaccaac 3000
atctacaaga tcctgctgct ccaggcctac agattccacg cctgcgtgct ccagctgccc 3060
ttccaccagc aggtgtggaa gaatcccacc ttcttcctga gagtgatcag cgacaccgcc 3120
agcctgtgct acagcatcct gaaggccaag aatgccggca tgagcctggg cgccaagggc 3180
gccgctggac ccctgcccag cgaggccgtg cagtggctgt gccaccaggc cttcctgctg 3240
aagctgacca gacacagagt gacctacgtg cccctgctgg gcagcctgag aaccgcccag 3300

acccagctga gcagaaagct gcctggcaca accctgaccg ccctggaggc agccgcaaac 3360

cccgccctgc ccagcgactt caagaccatc ctggactag 3399

<210> 11
<211> 248
<212> PRT
<213> Artificial Sequence

<220>
<223> amino acid sequence of tTA

<400> 11
Met Ser Arg Leu Asp Lys Ser Lys Val Ile Asn Ser Ala Leu Glu Leu
1 5 10 15

Leu Asn Glu Val Gly Ile Glu Gly Leu Thr Thr Arg Lys Leu Ala Gln
20 25 30

Lys Leu Gly Val Glu Gln Pro Thr Leu Tyr Trp His Val Lys Asn Lys
35 40 45

Arg Ala Leu Leu Asp Ala Leu Ala Ile Glu Met Leu Asp Arg His His
50 55 60

Thr His Phe Cys Pro Leu Glu Gly Glu Ser Trp Gln Asp Phe Leu Arg
65 70 75 80

Asn Asn Ala Lys Ser Phe Arg Cys Ala Leu Leu Ser His Arg Asp Gly
85 90 95

Ala Lys Val His Leu Gly Thr Arg Pro Thr Glu Lys Gln Tyr Glu Thr
100 105 110

Leu Glu Asn Gln Leu Ala Phe Leu Cys Gln Gln Gly Phe Ser Leu Glu
115 120 125

Asn Ala Leu Tyr Ala Leu Ser Ala Val Gly His Phe Thr Leu Gly Cys
130 135 140

Val Leu Glu Asp Gln Glu His Gln Val Ala Lys Glu Glu Arg Glu Thr
145 150 155 160

Pro Thr Thr Asp Ser Met Pro Pro Leu Leu Arg Gln Ala Ile Glu Leu
165 170 175

Phe Asp His Gln Gly Ala Glu Pro Ala Phe Leu Phe Gly Leu Glu Leu
180 185 190

Ile Ile Cys Gly Leu Glu Lys Gln Leu Lys Cys Glu Ser Gly Gly Pro
195 200 205

Ala Asp Ala Leu Asp Asp Phe Asp Leu Asp Met Leu Pro Ala Asp Ala
210 215 220

Leu Asp Asp Phe Asp Leu Asp Met Leu Pro Ala Asp Ala Leu Asp Asp
225 230 235 240

Phe Asp Leu Asp Met Leu Pro Gly
245

<210> 12
<211> 747
<212> DNA
<213> Artificial Sequence

<220>
<223> nucleotide sequence of tTA

<400> 12
atgtcaaggc tggataaaag caaagtgatt aactccgctc tggaactgct gaacgaagtc 60

ggcattgagg ggctgaccac acgcaagctg gcacagaagc tgggagtgga gcagcccacc 120

ctgtactggc acgtgaagaa caagcgcgcc ctgctggacg ccctggccat cgagatgctg 180

gatcggcacc acacacactt ctgccctctg gagggcgaga gctggcagga cttcctgcgg 240

aacaatgcca agagctttag atgtgccctg ctgtcccaca gggatggagc aaaggtgcac 300

ctgggcacca gaccaacaga gaagcagtac gagaccctgg agaaccagct ggccttcctg 360

tgccagcagg gcttttctct ggagaatgcc ctgtatgccc tgagcgccgt gggacacttc 420

accctgggat gcgtgctgga ggaccaggag caccaggtgg ccaaggagga gagagagaca 480

cctaccacag actccatgcc ccctctgctg aggcaggcca tcgagctgtt tgatcaccag 540

ggcgccgagc cagccttcct gtttggcctg gagctgatca tctgcggcct ggagaagcag 600

ctgaagtgtg agtctggagg accagcagat gccctggacg atttcgacct ggatatgctg 660

cccgccgacg ccctggacga ttttgatctg gacatgctgc ctgctgatgc cctggatgat 720

tttgacctgg atatgctgcc tggataa 747

<210> 13
<211> 199
<212> PRT
<213> Artificial Sequence

<220>
<223> amino acid sequence of PuroR

<400> 13
Met Thr Glu Tyr Lys Pro Thr Val Arg Leu Ala Thr Arg Asp Asp Val
1 5 10 15

Pro Arg Ala Val Arg Thr Leu Ala Ala Ala Phe Ala Asp Tyr Pro Ala
20 25 30

Thr Arg His Thr Val Asp Pro Asp Arg His Ile Glu Arg Val Thr Glu
35 40 45

Leu Gln Glu Leu Phe Leu Thr Arg Val Gly Leu Asp Ile Gly Lys Val
50 55 60

Trp Val Ala Asp Asp Gly Ala Ala Val Ala Val Trp Thr Thr Pro Glu
65 70 75 80

Ser Val Glu Ala Gly Ala Val Phe Ala Glu Ile Gly Pro Arg Met Ala
85 90 95

Glu Leu Ser Gly Ser Arg Leu Ala Ala Gln Gln Gln Met Glu Gly Leu
100 105 110

Leu Ala Pro His Arg Pro Lys Glu Pro Ala Trp Phe Leu Ala Thr Val
115 120 125

Gly Val Ser Pro Asp His Gln Gly Lys Gly Leu Gly Ser Ala Val Val
130 135 140

Leu Pro Gly Val Glu Ala Ala Glu Arg Ala Gly Val Pro Ala Phe Leu
145 150 155 160

Glu Thr Ser Ala Pro Arg Asn Leu Pro Phe Tyr Glu Arg Leu Gly Phe
165 170 175

Thr Val Thr Ala Asp Val Glu Val Pro Glu Gly Pro Arg Thr Trp Cys
180 185 190

Met Thr Arg Lys Pro Gly Ala
195

<210> 14
<211> 600
<212> DNA
<213> Artificial Sequence

<220>
<223> nucleotide sequence of PuroR

<400> 14
atgaccgagt acaagcccac ggtgcgcctc gccacccgcg acgacgtccc cagggccgta 60

cgcaccctcg ccgccgcgtt cgccgactac cccgccacgc gccacaccgt cgatccggac 120

cgccacatcg agcgggtcac cgagctgcaa gaactcttcc tcacgcgcgt cgggctcgac 180

atcggcaagg tgtgggtcgc ggacgacggc gccgcggtgg cggtctggac cacgccggag 240

agcgtcgaag cgggggcggt gttcgccgag atcggcccgc gcatggccga gttgagcggt 300

tcccggctgg ccgcgcagca acagatggaa ggcctcctgg cgccgcaccg gcccaaggag 360

cccgcgtggt tcctggccac cgtcggcgtc tcgcccgacc accagggcaa gggtctgggc 420

agcgccgtcg tgctccccgg agtggaggcg gccgagcgcg ccggggtgcc cgccttcctg 480

gagacctccg cgccccgcaa cctccccttc tacgagcggc tcggcttcac cgtcaccgcc 540

gacgtcgagg tgcccgaagg accgcgcacc tggtgcatga cccgcaagcc cggtgcctga 600

600

<210> 15
<211> 124
<212> PRT
<213> Artificial Sequence

<220>
<223> amino acid sequence of ZeoR

<400> 15
Met Ala Lys Leu Thr Ser Ala Val Pro Val Leu Thr Ala Arg Asp Val
1 5 10 15

Ala Gly Ala Val Glu Phe Trp Thr Asp Arg Leu Gly Phe Ser Arg Asp
20 25 30

Phe Val Glu Asp Asp Phe Ala Gly Val Val Arg Asp Asp Val Thr Leu
35 40 45

Phe Ile Ser Ala Val Gln Asp Gln Val Val Pro Asp Asn Thr Leu Ala
50 55 60

Trp Val Trp Val Arg Gly Leu Asp Glu Leu Tyr Ala Glu Trp Ser Glu
65 70 75 80

Val Val Ser Thr Asn Phe Arg Asp Ala Ser Gly Pro Ala Met Thr Glu
85 90 95

Ile Gly Glu Gln Pro Trp Gly Arg Glu Phe Ala Leu Arg Asp Pro Ala
100 105 110

Gly Asn Cys Val His Phe Val Ala Glu Glu Gln Asp
115 120

<210> 16
<211> 375
<212> DNA
<213> Artificial Sequence

<220>
<223> nucleotide sequence of ZeoR

<400> 16
atggccaagt tgaccagtgc cgttccggtg ctcaccgcgc gcgatgtggc cggagcggtc 60

gagttctgga ccgaccggct cgggttcagc cgggacttcg tggaggacga cttcgccggt 120

gtggtccggg acgacgtgac cctgttcatc agcgcggtcc aggaccaggt ggtgccggac 180

aacaccctgg cctgggtgtg ggtgcgcggc ctggacgagc tgtacgccga gtggtcggag 240

gtcgtgtcca cgaacttccg ggacgcctcc gggccggcca tgaccgagat cggcgagcag 300

ccgtgggggc gggagttcgc cctgcgcgac ccggccggca actgcgtgca cttcgtggcc 360

gaggagcagg actaa 375

<210> 17
<211> 20
<212> DNA
<213> Artificial Sequence

<220>
<223> GX535 Forward primer

```
<400>    17
ctgattgaca tggatcacga                                                   20


<210>    18
<211>    20
<212>    DNA
<213>    Artificial Sequence

<220>
<223>    GX534 Reverse primer


<400>    18
ctctcgtcga tgaactgctt                                                   20
```

## Claims

1. A recombinant lentiviral vector comprising a gene encoding a TNF-related apoptosis-inducing ligand (TRAIL) protein, and a cytosine deaminase (CD) protein.

2. The recombinant lentiviral vector according to claim 1, wherein the TRAIL protein is a polypeptide having the amino acid sequence of SEQ ID NO: 1.

3. The recombinant lentiviral vector according to claim 1, wherein the CD protein is a polypeptide having the amino acid sequence of SEQ ID NO: 3.

4. The recombinant lentiviral vector according to claim 1, which comprises one or two promoters.

5. The recombinant lentiviral vector according to claim 4, wherein the promoter is a cytomegalovirus (CMV), respiratory syncytial virus (RSV), human elongation factor-1 alpha (EF-1$\alpha$) or tetracycline response elements (TRE) promoter.

6. The recombinant lentiviral vector according to claim 1, which comprises an internal ribosome entry site (IRES).

7. A recombinant lentivirus comprising a gene encoding a TRAIL protein and a CD protein.

8. The recombinant lentivirus according to claim 7, wherein the lentivirus is obtained through the steps of:

   transforming a host cell with the lentiviral vector according to claim 1, a packaging plasmid and an envelope plasmid; and
   isolating the lentivirus from the transformed host cell.

9. A host cell transfected with the recombinant lentivirus according to claim 7.

10. The host cell according to claim 9, which is a mesenchymal stem cell.

11. A pharmaceutical composition for preventing or treating cancer comprising the recombinant lentivirus according to claim 7 as an active ingredient.

12. The pharmaceutical composition according to claim 11, wherein the cancer is selected from the group consisting of gastric cancer, colon cancer, breast cancer, lung cancer, non-small cell lung cancer, bone cancer, pancreatic cancer, skin cancer, head and neck cancer, melanoma, uterine cervix cancer, ovarian cancer, rectal cancer, endometrial cancer, Hodgkin's disease, a brain tumor, sarcoma, esophageal cancer, small bowel cancer, thyroid cancer, prostate cancer, leukemia, lymphoma, bladder cancer, a central nervous system tumor and a spinal cord tumor.

13. A pharmaceutical composition for preventing or treating cancer comprising the host cell according to claim 9 as an

active ingredient.

[Figure 1]

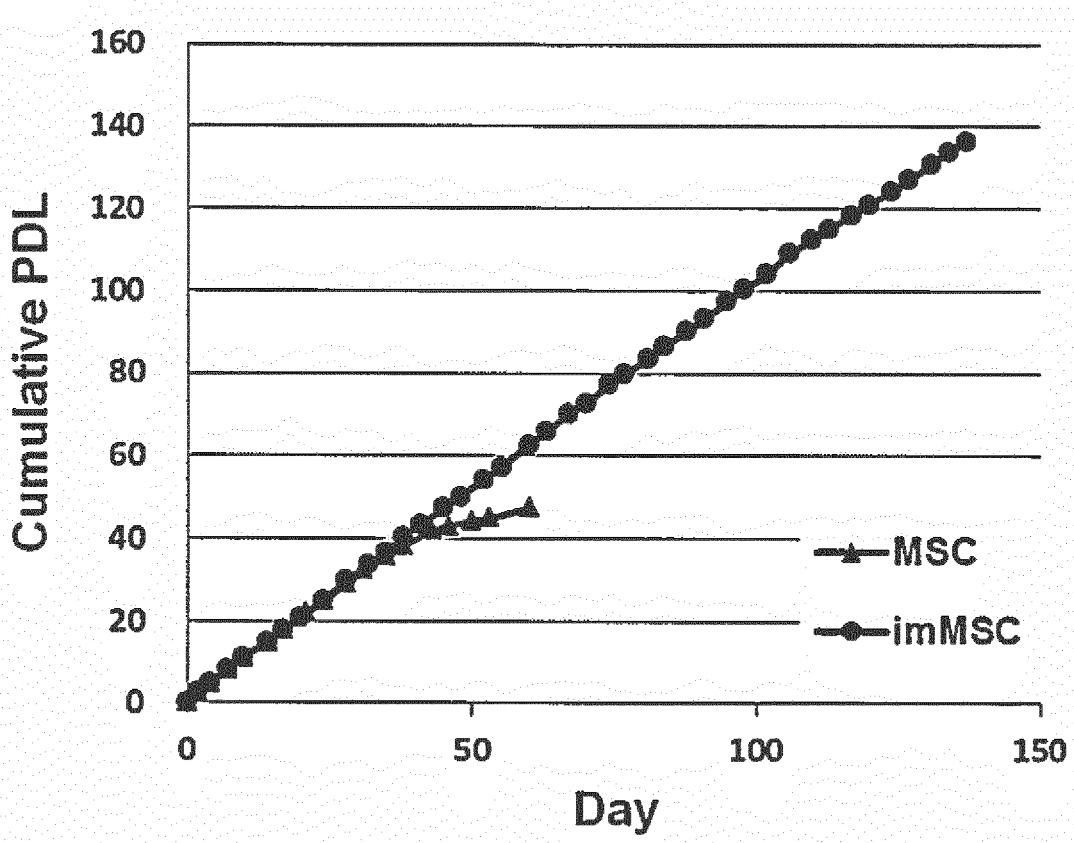


[Figure 2]

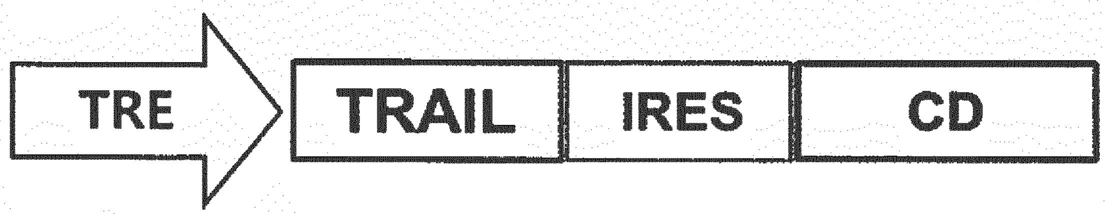

[Figure 3]

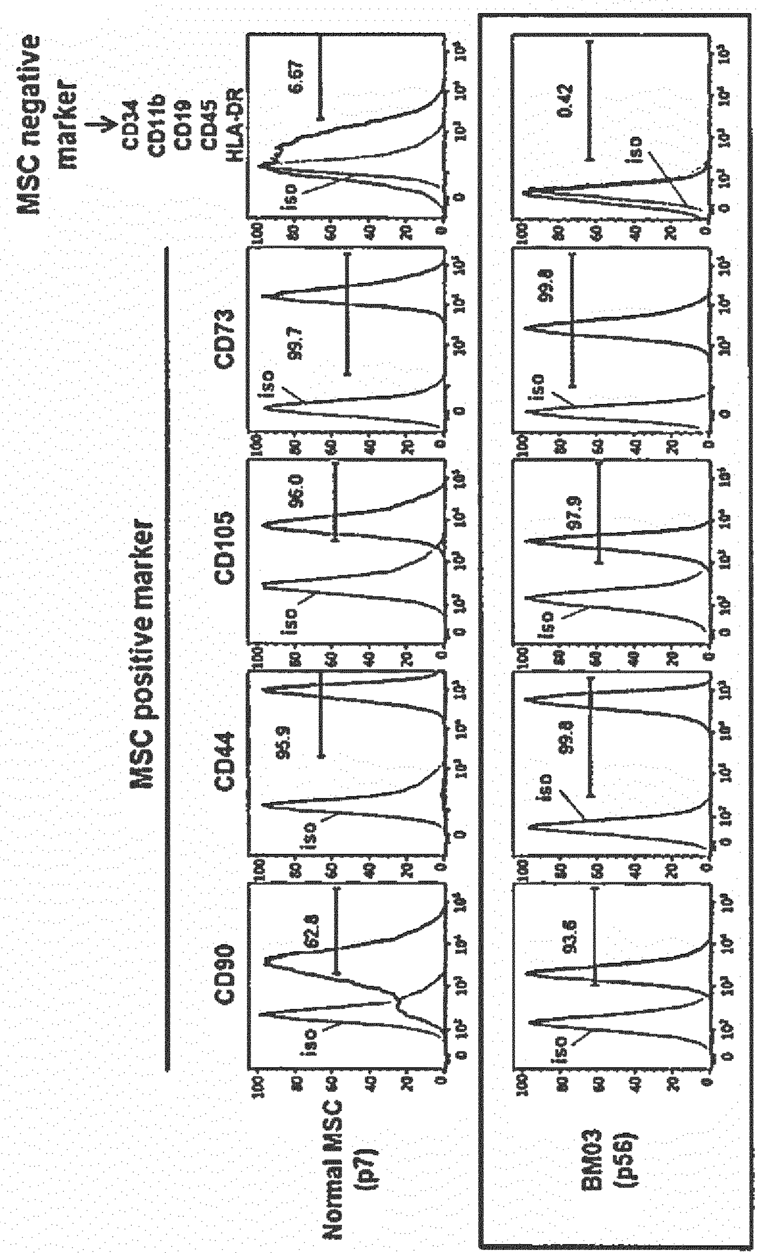
MSC negative marker
CD34
CD11b
CD19
CD45
HLA-DR
MSC positive marker
CD90
CD44
CD105
CD73
Normal MSC (p7)
BM03 (p56)
iso
62.8
95.9
96.0
99.7
6.67
93.6
99.8
97.9
99.8
0.42

[Figure 4]

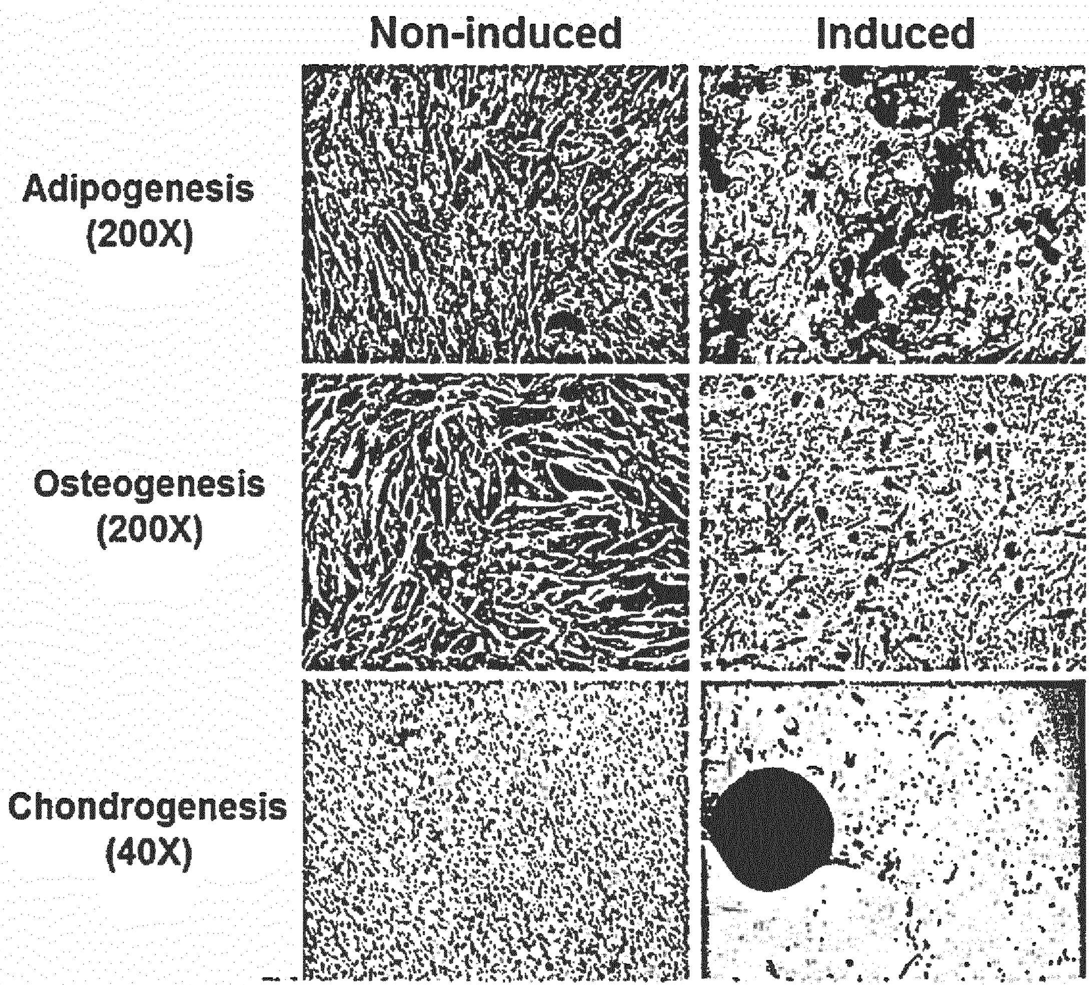
Non-induced
Induced
Adipogenesis
(200X)
Osteogenesis
(200X)
Chondrogenesis
(40X)

[Figure 5]

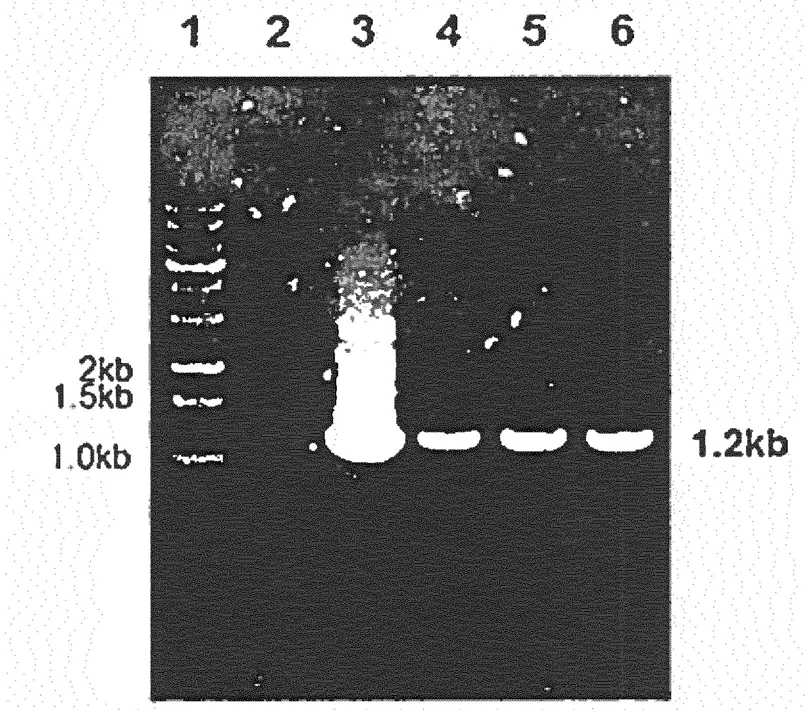


| | |
|---|---|
| Lane 1 | 1kb DNA ladder |
| Lane 2 | Negative control |
| Lane 3 | Positive control |
| Lane 4 | BM-03 Complete product 1 |
| Lane 5 | BM-03 Complete product 2 |
| Lane 6 | BM-03 Complete product 3 |

[Figure 6]

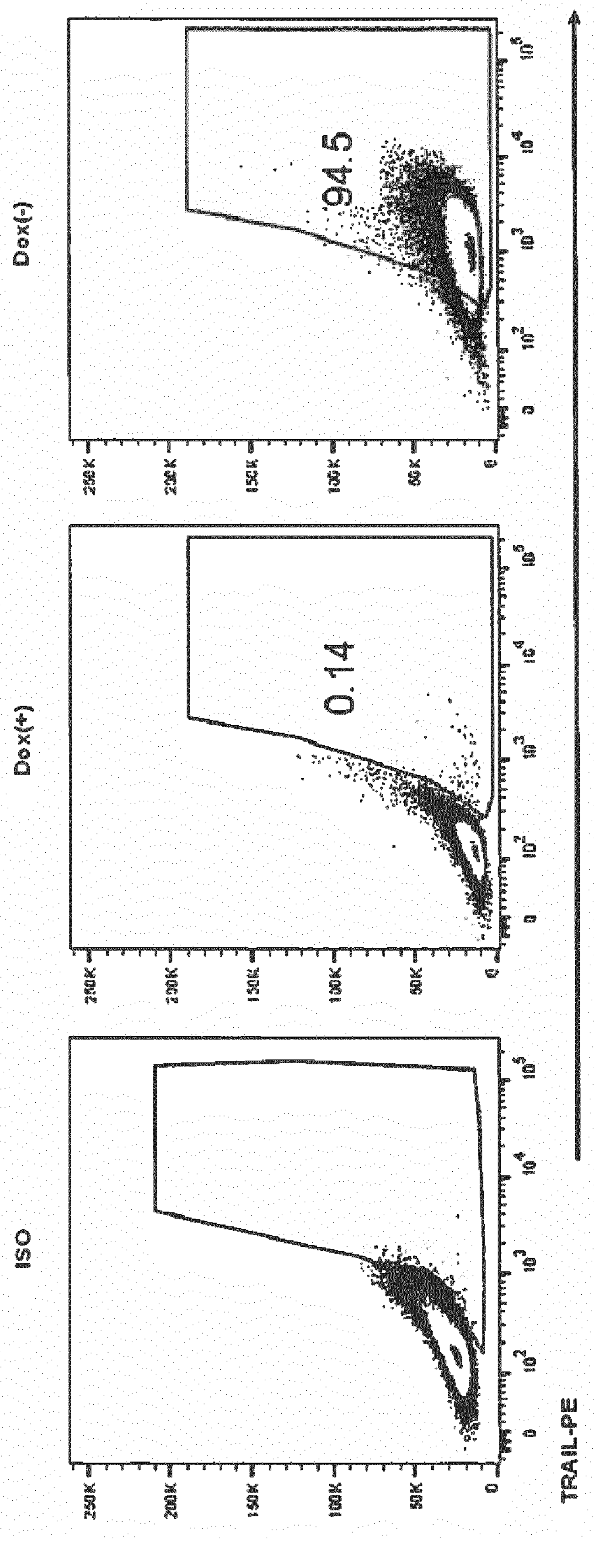

[Figure 7]

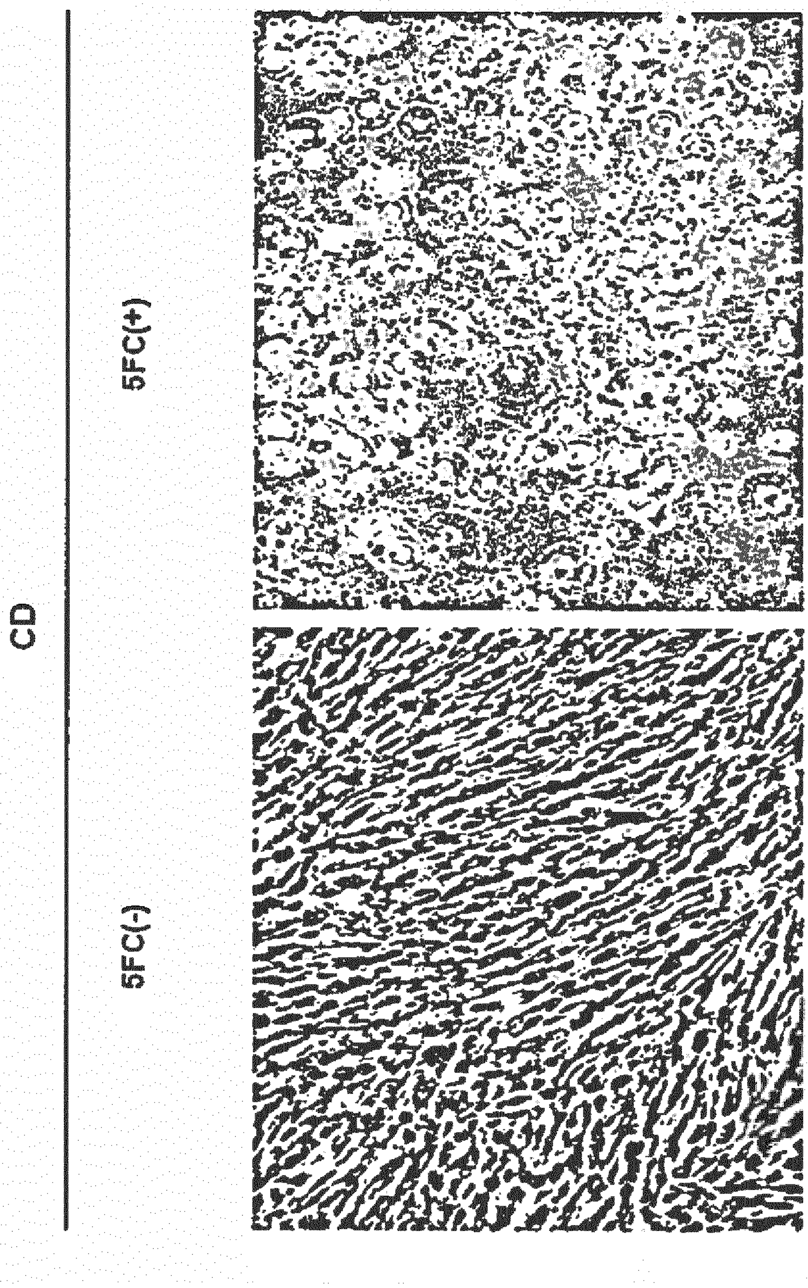

[Figure 8]

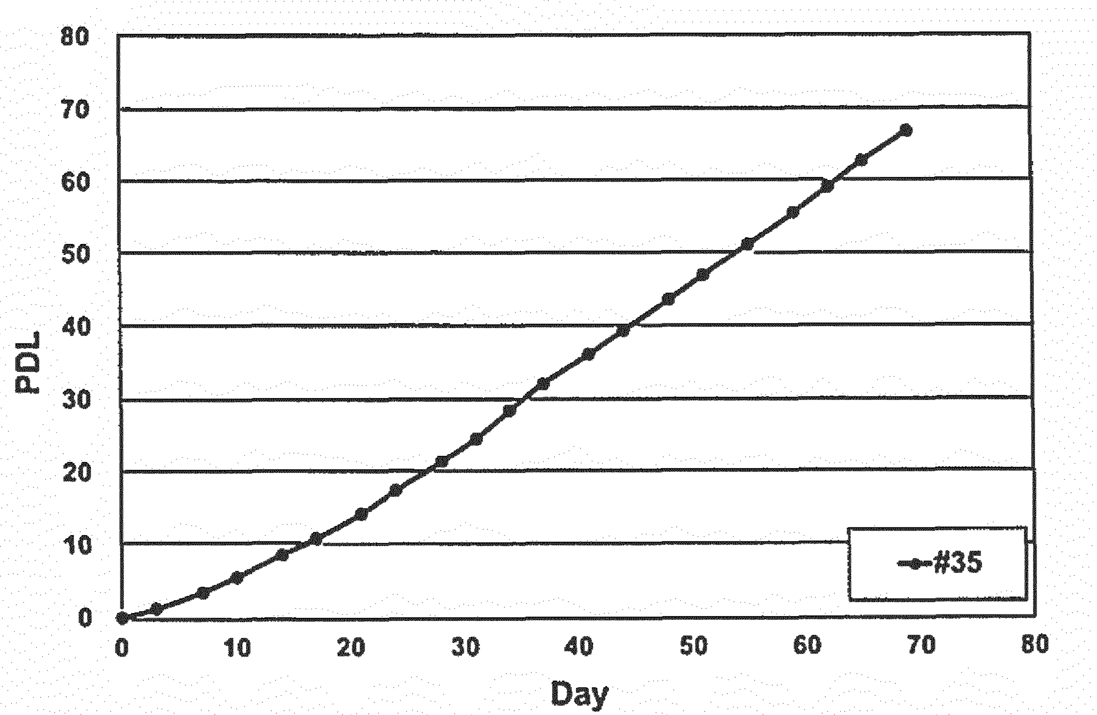


[Figure 9]

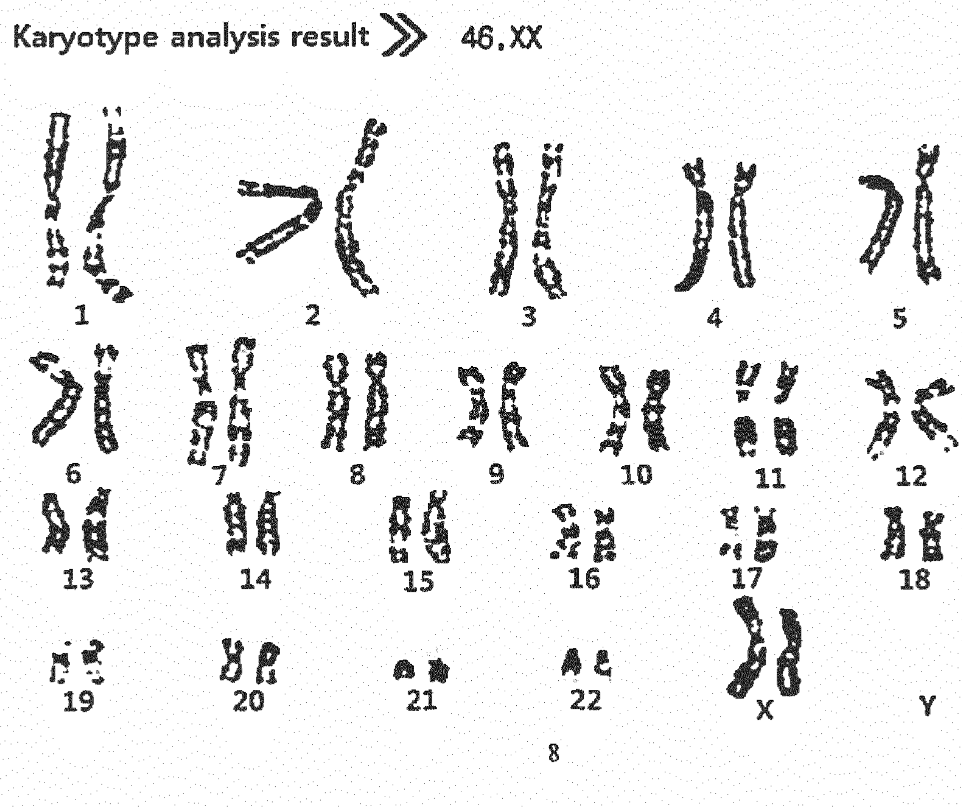

**INTERNATIONAL SEARCH REPORT**

International application No.
**PCT/KR2017/001308**

A. CLASSIFICATION OF SUBJECT MATTER

*C12N 15/86(2006.01)i, C07K 14/47(2006.01)i, C12N 9/10(2006.01)i, C12N 9/78(2006.01)i, C12N 5/0775(2010.01)i, A61K 35/28(2006.01)i, A61K 48/00(2006.01)i*

According to International Patent Classification (IPC) or to both national classification and IPC

B. FIELDS SEARCHED

Minimum documentation searched (classification system followed by classification symbols)

C12N 15/86; C12N 15/55; C07K 14/525; C12N 15/19; C12N 15/12; C12N 15/54; C12N 15/861; C07K 14/47; C12N 9/10; C12N 9/78; C12N 5/0775; A61K 35/28; A61K 48/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: TRAIL, cytosine deaminase, Lentivirus, mesenchymal stem cell, cancer treatment

C. DOCUMENTS CONSIDERED TO BE RELEVANT

| Category* | Citation of document, with indication, where appropriate, of the relevant passages | Relevant to claim No. |
|---|---|---|
| Y | KR 10-2013-0089214 A (POSTECH ACADEMY-INDUSTRY FOUNDATION et al.) 09 August 2013<br>See abstract; claims 1-3, 12-17; and paragraphs [0023], [0035]-[0050]. | 1-13 |
| Y | WENGER et al., "Specific Resistance upon Lentiviral TRAIL Transfer by Intracellular Retention of TRAIL Receptors", Cell Death & Differentiation, vol. 13, pp. 1740-1751 (2006)<br>See abstract; and page 1748. | 1-13 |
| A | US 5763223 A (WILEY et al.) 09 June 1998<br>See claims 1-24; and sequence no. 2 of columns 35-38. | 1-13 |
| A | US 6596533 B1 (ERBS et al.) 22 July 2003<br>See claims 1-12; and sequence no. 2 of columns 25-28. | 1-13 |
| A | WEI et al., "Cytosine Deaminase/5-fluorocytosine Gene Therapy and Apo2L/TRAIL Cooperate to Kill TRAIL-resistant Tumor Cells", Cancer Gene Therapy, vol. 14, pp. 640-651 (2007)<br>See the entire document. | 1-13 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 APRIL 2017 (26.04.2017) | **01 MAY 2017 (01.05.2017)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2017/001308**

| Patent document cited in search report | Publication date | Patent family member | Publication date |
|---|---|---|---|
| KR 10-2013-0089214 A | 09/08/2013 | CN 104160027 A | 19/11/2014 |
| | | CN 104160027 B | 22/06/2016 |
| | | EP 2811023 A1 | 10/12/2014 |
| | | JP 2015-506697 A | 05/03/2015 |
| | | US 2014-0369979 A1 | 18/12/2014 |
| | | WO 2013-115608 A1 | 08/08/2013 |
| US 5763223 A | 09/06/1998 | AU 5596599 A1 | 13/01/2000 |
| | | AU 708239 B2 | 29/07/1999 |
| | | AU 724826 B2 | 28/09/2000 |
| | | CA 2225378 A1 | 16/01/1997 |
| | | CA 2225378 C | 17/04/2012 |
| | | EP 0835305 A1 | 15/06/2005 |
| | | EP 0835305 B1 | 23/11/2005 |
| | | EP 1666591 A1 | 07/06/2006 |
| | | EP 1666591 B1 | 23/03/2011 |
| | | JP 11-508445 A | 27/07/1999 |
| | | JP 4435304 B2 | 17/03/2010 |
| | | KR 10-0510234 B1 | 25/11/2005 |
| | | KR 10-1004174 B1 | 24/12/2010 |
| | | US 2005-0158823 A1 | 21/07/2005 |
| | | US 2009-0118186 A1 | 07/05/2009 |
| | | US 2010-0323399 A1 | 23/12/2010 |
| | | US 6284236 B1 | 04/09/2001 |
| | | US 6521228 B1 | 18/02/2003 |
| | | US 7736637 B2 | 15/06/2010 |
| | | US 7972812 B2 | 05/07/2011 |
| | | WO 97-01633 A1 | 16/01/1997 |
| US 6596533 B1 | 22/07/2003 | AU 3335499 A | 08/11/1999 |
| | | AU 766911 B2 | 23/10/2003 |
| | | CA 2292882 A1 | 28/10/1999 |
| | | CA 2292882 C | 05/06/2012 |
| | | DE 69928745 T2 | 10/08/2006 |
| | | EP 0998568 A1 | 10/05/2000 |
| | | EP 0998568 B1 | 07/12/2005 |
| | | FR 2777570 A1 | 22/10/1999 |
| | | JP 2002-511769 A | 16/04/2002 |
| | | JP 4349660 B2 | 21/10/2009 |
| | | US 2003-0186415 A1 | 02/10/2003 |
| | | US 2006-0110363 A1 | 25/05/2006 |
| | | US 2010-0316610 A1 | 16/12/2010 |
| | | US 7049117 B2 | 23/05/2006 |
| | | US 7572438 B2 | 11/08/2009 |
| | | US 8211421 B2 | 03/07/2012 |
| | | WO 99-54481 A1 | 28/10/1999 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1585032
- US 5338678 A
- WO 9616183 A
- WO 9954481 A